(19)

**Europäisches Patentamt**
**European Patent Office**
**Office européen des brevets**

(11) **EP 4 651 150 A1**

(12) **EUROPEAN PATENT APPLICATION**

(43) Date of publication:
**19.11.2025 Bulletin 2025/47**

(21) Application number: **24176611.2**

(22) Date of filing: **17.05.2024**

(51) International Patent Classification (IPC):
**G16H 50/20** (2018.01)    **G16H 50/30** (2018.01)
**G16H 50/70** (2018.01)

(52) Cooperative Patent Classification (CPC):
**G16H 50/20; G16H 50/30; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Fig, Ricardo Damián**
**Newbury RG142FY (GB)**

(72) Inventor: **Fig, Ricardo Damián**
**Newbury RG142FY (GB)**

(74) Representative: **ABG Intellectual Property Law, S.L.**
**Avenida de Burgos, 16D**
**Edificio Euromor**
**28036 Madrid (ES)**

(54) **HEALTH ALARMS SYSTEM**

(57)    The present invention is directed to a system for issuing alarms related to the health of a subject. More particularly, the system comprises a processor with an Artificial Intelligence module configured to determine the probability of the subject to suffer from a disease and to emit an alarm in case such probability is above a pre-defined value.

EP 4 651 150 A1

**Description**

**TECHNICAL FIELD OF THE INVENTION**

The present invention is directed to a system for issuing alarms related to the health of a subject. More particularly, the system comprises a processor with an Artificial Intelligence module configured to determine the probability of the subject to suffer from a disease and to emit an alarm in case such probability is above a predefined value.

**BACKGROUND OF THE INVENTION**

The healthcare industry is focused on the early detection of health issues and the individualization of treatments.

An early detection of health issues allows the detection of diseases or, in case the patient suffers from a disease, the detection of a deterioration of the same. The individualization of treatments allows the treatment to be as effective as possible for a particular patient. Both this early detection and this individualization of treatment are essential for the healthcare providers to be able to timely intervene, for preventing complications and potentially saving lives.

There are several factors that prevent or hinder the achievement of these aims, such as the long waiting lists to be attended by healthcare providers, the human errors, the difficulties in allocating the resources (healthcare providers and equipment), and problems of limited access to healthcare for patients who live in remote areas or have mobility issues.

To these factors are added the technical problems associated with data security and privacy, given the strict confidentiality of healthcare data, as well as the integration of such data with existing medical devices and electronic health record (EHR) systems. An incorrect integration leads to having fragmented healthcare data, that is, the healthcare and personal patient information is scattered across different systems so it may not be easily accessible to healthcare providers. In this way, the healthcare providers will not be able to have a comprehensive view of a patient's medical history and status.

There is thus a need for a system for the early detection of health issues that can solve technical problems related to the access to healthcare and to data integration with existing medical devices and EHR systems, such system being able to improve the resources allocation and to reduce the human errors at the same time.

**SUMMARY OF THE INVENTION**

The present invention provides a solution for the aforementioned problems by a system for issuing alarms related to the health of a subject according to claim 1. In dependent claims, preferred embodiments of the invention are defined.

In a first inventive aspect, the invention provides a system for emitting alarms related to the health of a subject, the system comprising a processor configured to:

- receive at least one health parameter of a subject, wherein one or more of the at least one health parameter is measured by at least one sensor, and
- receive at least one personal parameter of the subject,

wherein the processor comprises an Artificial Intelligence, AI, module trained to generate a plurality of disease models as a result of the training, the training being carried out with:

- at least one health parameter and/or at least one personal parameter of subjects that suffer from at least one of the diseases of the disease models; and
- at least one health parameter and/or at least one personal parameter of subjects that do not suffer from said disease;

wherein each disease model is based on a neural network comprising:

◦ an input layer comprising N nodes, N being the number of health parameters and personal parameters used for the training of the disease model;
◦ three hidden layers, each one comprising an activation function and a plurality of nodes configured to receive a plurality of inputs; and
◦ an output layer with a single node representing the probability of the subject to suffer from the disease; and

wherein the output of each node of each hidden layer of the neural network is the result of applying its corresponding activation function to a weighted sum of its corresponding inputs;
and wherein the AI module is configured to carry out the following steps:

a) using the at least one health parameter and/or the at least one personal parameter as input for one or more of the disease models;

b) determining the probability of the subject to suffer from a disease; and

c) emitting an alarm if the probability exceeds a determined probability value.

Throughout all this document, "subject" will be understood as a person who may become ill with a disease or a patient that suffers from a disease.

Throughout all this document, "health parameter" will be understood as a parameter that provides information of a subject health condition, and may include physiological data and/or behavioral data; for example, the heart rate of the subject, the sleep pattern of the subject or the level of oxygen in the blood of the subject.

In an embodiment, the at least one health parameter comprises at least one subject vital sign. Throughout all this document, "vital sign" will be understood as a parameter that reflects the essential functions of a subject's body: the heart rate, the respiratory rate, the temperature and the blood pressure.

In a particular embodiment, the at least one health parameter is one or more from:

- type of chest pain experienced by the subject,
- level of blood pressure at resting mode,
- serum cholesterol,
- maximum heart rate experienced by the subject,
- angina induced by exercise,
- ST-depression induced by exercise in comparison with the resting mode,
- subject suffering a diabetes disease,
- subject suffering a depression disease,
- subject with APOE $\varepsilon$3 allele,
- level of oxygen in blood,
- heart rate,
- time elapsed between the subject wears a wearable device and a magnetic resonance imaging study is performed or between a magnetic resonance imaging study is performed and the subject puts on the wearable device,
- level of alcohol in blood,
- body temperature,
- weight,
- sleep pattern,
- steps movements pattern,
- clinical dementia rating, that indicates the severity of dementia symptoms,
- mini mental state examination, that indicates the degree of cognitive function,
- normalized whole brain volume,
- estimated total intracranial volume,
- atlas scaling factor,
- heart rate variability,
- electrodermal activity,
- respiratory rate and respiratory patterns,
- level of blood pressure,
- activity levels,
- social interaction pattern,
- substance use or abuse history,
- self-reported symptoms of anxiety and/or depression,
- history of mental health diagnoses or treatments,
- body mass index,
- number of binge episodes per week,
- number of purging episodes per week,
- body dissatisfaction score, that indicates the score of self-reported body dissatisfaction,
- eating disorder examination questionnaire score, that indicates the score of the eating disorder symptoms according to a standard,
- depression score, that indicates the score from a depression assessment scale,
- anxiety score, that indicates the score from an anxiety assessment scale, and
- eating disorder diagnosis.

At least one of the health parameters are measured by at least one sensor. In an embodiment, the sensor is part of the system. In an embodiment, the sensor is part of a wearable device.

In an embodiment, one or more of the health parameters is a symptom description provided by the subject, for example the type of chest pain experienced by the subject, the self-reported symptoms of anxiety or the body dissatisfaction score.

In an embodiment, one or more of the health parameters comprises behavioral data, for example, the social interaction pattern of the subject.

In an embodiment, one or more of the health parameters reflects lifestyle factors, for example, substance use or abuse history of the subject.

In an embodiment, one or more of the health parameters is a subject's disease previously diagnosed, for example, a subject suffering from a diabetes disease or a depression disease.

In an embodiment, one or more of the health parameters is obtained from standard questionnaires answered by the subject, for example, the eating disorder examination questionnaire score.

In an embodiment, one or more of the health parameters are based on medical imaging studies, for example, the normalized whole brain volume or the atlas scaling factor.

In an embodiment, the processor of the system receives one or more of the health parameters from a sensor or sensors in real-time or at regular intervals.

In an embodiment, the processor of the system receives one or more of the health parameters from a medical database and/or from the subject and/or from a caregiver and/or from a healthcare provider.

Throughout all this document, "personal parameter" will be understood as a parameter that provides information of the subject's personal data; for example, the age or the sex of the subject.

In an embodiment, the processor of the system receives the at least one personal parameter from a medical database and/or from the subject and/or from a caregiver and/or from a healthcare provider.

The processor of the system is configured to receive at least one health parameter, one or more of said health parameters being measured by the at least one sensor, and at least one personal parameter, for example, from the clinical history of the subject, electronic health records (EHR) systems, subject's self-reports, or a profile of the subject registered in the system previously.

In an embodiment, the transmission of the health and/or personal parameters is performed through a mobile application or a cloud-based platform.

The processor comprises an AI module that is trained for generating disease models. Such training considers N health parameters and/or personal parameters of real subjects that suffer from at least one of the diseases represented by the disease models and of subjects without said disease/s. Such data is taken, for example, from medical databases. In an embodiment N is greater than 1.

Each disease model is a neural network with an input layer with N nodes, three hidden layers and an output layer.

Each hidden layer comprises an activation function and a plurality of nodes that receive a plurality of inputs.

Throughout all this document, "activation function" will be understood as a mathematical function that defines the output of a node based on the node's inputs. In particular, the output of each node of the neural network is the result of applying its corresponding activation function to a weighted sum of its corresponding inputs.

The first hidden layer receives, as its inputs, the outputs of the input layer; the second hidden layer receives, as its inputs, the outputs of the first hidden layer; and the third hidden layer receives, as its inputs, the outputs of the second hidden layer.

The output layer receives, as its inputs, the outputs of the third hidden layer. The output layer comprises a single node that provides the probability of the subject to suffer from the disease of the specific disease model.

Once the AI module has generated the disease model, said module is configured to determine the probability of the subject to suffer from a disease using the at least one health parameters and/or the at least one personal parameters received by the processor as the input of the disease models. In case the probability determined exceeds a predetermined probability value, the AI module emits an alarm.

In an embodiment, for at least one of the disease models, the health parameters and/or the personal parameters used by the AI module for determining the probability of the subject to suffer from the disease are at least part of the health parameters and/or personal parameters used by the AI module for generating the disease model. Preferably, all the health parameters and/or personal parameters used by the AI module for determining such probability are all the health parameters and/or personal parameters used by the AI module for generating the disease model.

In an embodiment, the processor is configured for receiving at least one health parameter of the subject measured by a sensor and the AI module is configured for comparing the value of the at least one health parameter with a predefined range of values. The AI module is further configured for emitting an alarm if the value measured is outside the predefined range of values.

In an embodiment, the range of values is defined by a healthcare provider according to a standard and the system is configured to store such range of values, for example, in an internal database. In an embodiment, the range of values is modified by the healthcare provider according to a disease of the subject and/or according to the symptoms of the subject and/or according to the health and/or personal parameters of the subject.

In an embodiment, the processor is configured for receiving at least one health parameter of the subject measured by a sensor over time and the AI module is configured for comparing a current value of the at least one health parameter with a previous value of the at least one health parameter measured. The AI module is further configured for emitting an alarm if the current value measured suddenly changes in comparison with the previous value.

In an embodiment, the alarm is emitted by the system towards the healthcare providers, and/or caregivers and/or a member of the subject's family and/or any other contact person and/or the subject, in order to inform about the health condition of the subject.

Throughout all this document, "healthcare provider" will be understood as any member of the healthcare team who could attend the subject, e.g. doctors, nurses or psychologists.

The system of the first inventive aspect uses AI neural networks with predictive modeling techniques to forecast future health outcomes, which leads to healthcare providers to anticipate exacerbations of chronic conditions and prevent adverse events.

Thanks to the system of the invention, healthcare providers can remotely monitor subject's health status following up the health and personal parameters to track the appearance and progress of a disease, and to intervene as needed without requiring in-person visits.

More advantages of the system of the invention are listed below.

- With the help of the AI prediction, healthcare provides can make informed decisions, avoiding human errors, to anticipate any disease that the subject may be developing.
- The health parameters can be continuously monitored, for example using the sensors of wearable devices. In this way, the health parameters obtained can be provided in real-time to the system, what leads to a better-informed decision-making and personalized care.
- The continuous monitoring allows to detect subtle changes in a subject's condition early, which may indicate the onset of a disease. This early detection can lead to timely interventions, preventing complications, reducing hospitalizations, improving patient outcomes and potentially saving lives.
- Remote subject monitoring is quite relevant for subjects with limited access to healthcare who live in remote areas or have mobility issues, especially those with chronic illnesses or recovering from surgeries. Subjects can be monitored from the comfort of their homes, reducing hospital admissions and the burden on healthcare facilities.
- The continuous monitoring also helps to optimize resource allocation by prioritizing care based on subject's severity and needs. This reduces the strain on healthcare providers and resources, and ensures that the subjects receive timely care.
- Automation in subject monitoring also decreases the risk of transcription errors and misinterpretation of data (human errors).

In an embodiment, the system is configured to transmit data to and receive data from at least one existing medical device and/or at least one electronic health record (EHR) system. This solves the problem of fragmented data in healthcare, where patient information is scattered across different systems and may not be easily accessible to healthcare providers. This allows the system to provide a comprehensive view of a patient's medical history and status.

In an embodiment, to address the technical problem of securing patient data, the system implements subject's data encryption, and/or access controls, and/or compliance with healthcare privacy regulations (e.g., HIPAA).

In an embodiment, the system comprises a dashboard to allow the healthcare providers to have continuous access to real-time subject's health parameters and/or personal parameters, and/or the AI prediction.

In this way, the healthcare providers can detect abnormalities in the subject's parameters so that they can react in time if needed. For example, if a subject diagnosed with a heart disease suddenly has a drastic rise or fall in his/her heart rate, the healthcare providers can warn the emergency services.

In an embodiment, the system is configured to transmit data to and receive data from clinical decision support tools used by healthcare providers. Advantageously, the system offers real-time guidance and decision support to the healthcare providers.

In an embodiment, the system is configured for feeding the AI module automatically with information from new subjects suffering from one or more diseases and with medical literature updates to train the AI module. Advantageously, the disease models are refined and the accuracy and effectiveness of the predictions over time is improved.

In an embodiment, the AI module is further trained to generate a plurality of disease severity models as a result of the training, the training being carried out with at least one health parameter and/or at least one personal parameter of subjects that suffer from different degrees of severity of a disease of the disease severity models;

wherein each disease severity model is based on a neural network comprising:

◦ an input layer comprising M nodes, M being the number of health parameters and personal parameters used for the training of the disease severity model;

◦ three hidden layers, each one comprising an activation function and a plurality of nodes configured to receive a plurality of inputs; and

◦ an output layer with a single node representing the severity of the disease of the subject; and

wherein the output of each node of each hidden layer of the neural network is the result of applying its corresponding activation function to a weighted sum of its corresponding inputs;

and wherein the AI module is further configured to carry out the following steps:

1. using the at least one health parameter and/or the at least one personal parameter as input for one or more of the disease severity models;

2. determining a disease severity index for the disease of the subject; and

3. emitting an alarm if the disease severity index exceeds a determined severity value.

In this embodiment, the AI module is trained for generating disease severity models. Such training considers M health parameters and/or personal parameters of real subjects that suffer different degrees of severity of one or more of the diseases represented by the disease severity models. Such data is taken, for example, from medical databases. In an embodiment M is greater than 1.

In an embodiment, the disease severity index is a numerical value that indicates the degree of severity of a disease.

Each disease severity model is a neural network with an input layer with M nodes, three hidden layers and an output layer.

Each hidden layer comprises an activation function and a plurality of nodes that receive a plurality of inputs.

The first hidden layer receives, as its inputs, the outputs of the input layer; the second hidden layer receives, as its inputs, the outputs of the first hidden layer; and the third hidden layer receives, as its inputs, the outputs of the second hidden layer.

The output layer receives, as its inputs, the outputs of the third hidden layer. The output layer comprises a single node that provides the disease severity index for the disease of the subject.

Once the AI module has generated the disease severity model, said module is configured to determine the disease severity index for the disease of the subject using the at least one health parameter and/or the at least one personal parameter received by the processor as the input of the disease severity models. In case the disease severity index determined exceeds a predetermined severity value, the AI module emits an alarm.

In an embodiment, for at least one of the disease severity models, the health parameters and/or the personal parameters used by the AI module for determining the severity of the disease of the subject are at least part of the health parameters and/or personal parameters used by the AI module for generating the disease severity model. Preferably, all the health parameters and/or personal parameters used by the AI module for determining such severity are all the health parameters and/or personal parameters used by the AI module for generating the disease severity model.

Advantageously, the system can continuously estimate the severity of the disease of the subject so that, when the severity is over a predefined threshold, an alarm is emitted. For example, if a subject with a heart disease reaches a certain degree of severity for the disease, the AI module emits an emergency call for the emergency services as such degree of severity can be indicating a heart attack.

Additionally, by comparing the severities estimated by the system over time, healthcare providers can detect a deterioration of a subject's disease and, optionally, take some actions; for example, a modification of the subject's medication plan adapted to the deteriorated disease.

The system is also useful in critical care units and during surgeries. Continuous monitoring of the subject's health parameters provide real-time data on vital signs and can trigger alarms in case a degree of severity is reached, enabling immediate attention from healthcare professionals.

In a particular embodiment, the processor is configured to select the health parameters and/or the personal parameters used to train the AI module for generating each disease model, wherein the selection is carried out using a plurality of decision trees, each decision tree comprising a plurality of layers, and each layer comprising at least one parent node, at least one left child node and at least one right child node, and wherein the processor is configured to carry out the following steps:

- using the health parameters and/or the personal parameters as the inputs of the plurality of decision trees;

- determining an importance value of each health parameter and each personal parameter for each layer of a decision tree as the decrease in node impurity weighted by the probability of reaching the at least one parent node of the layer; wherein

○ the probability of reaching the parent node of the layer is determined as the number of health parameters and personal parameters that reach said parent node divided by the total number of health parameters and personal parameters used to train the AI module; and
○ the decrease in node impurity is determined as follows:

$$Decrease\ node\ impurity = I_p - \left( \frac{N_{left}}{N_{right}} \cdot I_{left} + \frac{N_{right}}{N_{left}} \cdot I_{right} \right)$$

being:

$N_{left}$ and $N_{right}$ the total number of health parameters and personal parameters in the at least one left and the at least one right child nodes of the layer, respectively, and
$I_p$, $I_{left}$ and $I_{right}$ are, respectively, the impurities of the at least one parent node, the at least one left child node and the at least one right child node; the impurities being calculated with an impurity measure, preferably a Gini impurity measure or an entropy measure;

- determining a final importance value for each health parameter and each personal parameter as the importance value of said health parameter or personal parameter calculated for the last layer of the decision tree; and
- selecting the health parameters and/or personal parameters to be used to train the AI module as the ones with a final importance value over a predefined threshold.

As mentioned before, the AI module is trained to generate the disease models. Such training uses one or more health and/or personal parameters of subjects that suffer from at least one of the diseases and of subjects that do not suffer from said disease/s. However, not all the health and/or personal parameters are suitable for all the diseases but rather a selection of them is recommendable in order to obtain the best results in the subsequent disease predictions.
In this embodiment, for each disease, the selection of parameters is performed by means of decision trees. Throughout all this document, "decision tree" will be understood as a decision support hierarchical model that uses a tree-like model of decisions and their possible consequences.
With the decision trees, a final importance value is calculated for each health parameter and each personal parameter. The higher the final importance value, the most relevant is this parameter for a specific disease.
The selected parameters are those that reach a predefined threshold of final importance value. In an embodiment, such threshold is selected for each disease.
In an embodiment wherein the AI module is trained to generate a plurality of disease severity models, the processor is configured to select the health parameters and/or the personal parameters used to train the AI module for generating each disease severity model, wherein the selection is carried out using a plurality of decision trees as defined above.
In a particular embodiment, the processor is configured to process the health parameters and/or the personal parameters used to train the AI module before the training process for the disease models generation, the processor being configured to carry out the processing of the health parameters and/or the personal parameters for each disease performing the following steps:

- creating a table wherein each column represents a health parameter or a personal parameter and each row represents a different subject suffering from the disease;
- giving a predetermined numerical format to the values of the table;
- replacing the values of the table that do not present a numerical value with the mean of its column's values;

- identifying the outlier values of each column, preferably using a Z-score algorithm; and
- eliminating the row or rows in which the outlier values are located;
  or
- creating a table wherein each row represents a health parameter or a personal parameter and each column represents a different subject suffering from the disease;
- giving a predetermined numerical format to the values of the table;
- replacing the values of the table that do not present a numerical value with the mean of its row's values;
- identifying the outlier values of each row, preferably using a Z-score algorithm; and
- eliminating the column or columns in which the outlier values are located.

This embodiment describes a process for preparing the health and/or personal parameters before training the AI module for the disease models generation. The rows and columns of the table resulting from this process are interchangeable.

Firstly, the table is created with the information of a plurality of subjects suffering from a disease, the information being separated in health and/or personal parameters.

Then, a format conversion takes place so that a predetermined numerical format is given to all the values of the table. For example, integer or float values.

After the format conversion, some of the table values could present a value that is not numerical; that is, they are not-a-number (NaN). In such cases, the value is replaced by the mean of the column or row's values.

Finally, outlier values are detected so that the data corresponding to the subject presenting this value is deleted from the table (column or row of such subject). Throughout all this document, "outlier value" will be understood as an extreme value that stands out significantly from the overall pattern of values in a dataset.

In an embodiment, the identification of outlier values is performed by means of a Z-score algorithm.

This data cleaning and formatting process allows to train the AI module with quality data so that the subsequent predictions are more precise.

In an embodiment wherein the AI module is trained to generate a plurality of disease severity models, the processor is configured to process the health parameters and/or the personal parameters used to train the AI module before the training process for the disease severity models generation, the processor being configured to carry out the processing of the health parameters and/or the personal parameters for each disease severity model performing the previously mentioned steps.

In a particular embodiment, the system comprises at least one sensor. In an embodiment, the system comprises a plurality of sensors. In an embodiment, one or more of the at least one sensor is configured to measure one or more of the at least one health parameter of the subject.

In a particular embodiment, the system comprises at least one sensor comprising a three axis gyroscope and a three axis accelerometer and the AI module is further configured for

- detecting if the subject has fainted or has fallen down by analyzing the angular velocity measured by the three axis gyroscope and the acceleration measured by the three axis accelerometer, and
- emitting an alarm in case the subject has fainted or has fallen down.

According to this embodiment, the system of the invention can also detect, by analyzing the information coming from a three axis gyroscope and a three axis accelerometer, if a subject has fainted or has fallen down. In such cases, the system emits an alarm to inform, for example, the healthcare providers, the caregivers or the family about the fall or the fainting of the subject.

The AI module of the system can detect falls and fainting by integrating the information of the three-axis gyroscope and the three-axis accelerometer; that is, the AI module takes into account the measurements of the six axes of the sensors taken at a plurality of times. The AI module estimates the position of the patient according to the measurements of the six axes of the sensors at the plurality of times and, in case the position estimated by the AI module suffers a sudden change during a specific period of time (for example, in less than 4 seconds), the AI module detects that the subject has fainted or has fallen down. Consequently, the AI module emits an alarm.

This functionality is especially relevant for elderly patients and patients with heart diseases, whose fall or fainting must be attended urgently.

In an embodiment, the AI module takes into account the measurements of the six axes of the sensors taken every 10 ms.

In a particular embodiment, the system comprises at least one sensor configured for tracking the location of the subject and the AI module is further configured to emit an alarm if the subject is out of a predefined area.

In this embodiment, the system of the invention can also track the location of the subject continuously. Thanks to the location information, the AI module can emit an alarm if the patient is out a predefined area, for example, his/her house or his/her hospital room. In such cases, the system emits an alarm to inform, for example, the healthcare providers, the caregivers, a contact person or the family about the subject's location.

In an embodiment, GPS and/or Bluetooth and/or beacon and/or geofencing technologies are used to track the subject's location.

This functionality is especially relevant for subjects that suffer from schizophrenia, dementia or Alzheimer, as they can easily become disoriented.

In a particular embodiment, the AI module is further configured for:

- recommending a personalized treatment plan according to the probability of the subject to suffer from a disease; and/or
- associating a subject with at least one specific healthcare provider according to the probability of the subject to suffer from a disease.

In this embodiment, the system provides further functionalities.

On the one hand, the AI module of the system can recommend a specific treatment for the subject according to the probability of being suffering the disease.

In an embodiment, the system comprises a database with a plurality of treatments intended for treating a specific disease. In this way, when the AI module emits an alarm when the probability of the subject to suffer from a disease exceeds a predetermined probability value, the AI module selects the treatment for this disease from the system's database.

Advantageously, the system allows to individualize the treatment for each subject, allowing the treatment to be as effective as possible and reducing the human errors.

In an embodiment, the treatment comprises medication plans, and/or physiotherapy plans, and/or psychotherapy plans, and/or rehabilitation plans, and/or surgical interventions, and/or lifestyle modifications.

In an embodiment, the AI module is further configured for providing explanations and justifications for the recommended treatment plans, highlighting the underlying rationale, evidence-based guidelines, and factors influencing the decision-making process. Advantageously, this enhances transparency and trust in the AI recommendations.

In an embodiment, the system is configured to receive reports (e.g. from the healthcare providers), wherein the reports indicate how the subject responded to at least one previously prescribed treatment, for example, to a previous medication plan. The reports can also include the symptoms that the subject manifested when the subject followed the prescribed treatment plan. The AI module is further configured to select a specific treatment plan for the subject taking into account the reports.

For example, if a subject that was following a previously prescribed medication plan has nausea and vomiting, and the healthcare provider has included such symptoms into a report, the AI module takes into account such symptoms to recommend a treatment plan that does not include the previously prescribed medication plan. In this way, the system avoids the negative response of the subject to such medication plan.

In an embodiment, the system is configured to receive reports (e.g. from the healthcare providers), wherein the reports indicate how the subject responds to the recommended treatment, for example, to a medication plan. The reports can also include the symptoms that the subject manifests when the subject is following the recommended plan. The AI module is further configured to improve the treatment plans for the subject taking into account the reports.

For example, if a subject was following a recommended treatment plan that comprises a medication plan, such medication plan leading the subject to suffer nausea and vomiting, and the healthcare provider has included such symptoms into a report, the AI module takes into account such symptoms to improve the treatment plan so that it does not include the previously recommended medication plan. In this way, the system avoids the negative response of the subject to such medication plan.

In an embodiment, the system is configured to transmit data to and receive data from clinical decision support tools used by healthcare providers. Advantageously, in this embodiment the system offers real-time guidance and decision support to the healthcare providers, who can make informed decisions aligned with the personalized treatment recommendations.

In an embodiment, the AI module of the system is further configured for generating collaborative treatment plans to be followed by both healthcare providers and caregivers. In an embodiment, the collaborative treatment plans take into account the subject's responses to previously prescribed treatment plans. Advantageously, such collaborative treatment plans allow caregivers and healthcare providers to provide person-centered care.

In an embodiment, the system is configured for feeding the AI module automatically with information from new subjects suffering from one or more diseases, treatment outcomes, and medical literature updates to train the AI module, in order to refine and improve the accuracy and effectiveness of the treatment recommendations over time.

In an embodiment, the system is configured to provide access to online support groups for caregivers and family members so that they can be in contact with the healthcare providers.

In an embodiment, the system is configured to provide access to the subject's family members, and/or caregivers and/or contact persons to relevant information about the subject's condition and to receive updates.

In addition or alternatively, the AI module of the system can associate at least one specific healthcare provider to the subject according to the probability of being suffering the disease. For example, in case of a mental health disease, the AI module can associate psychologists and psychiatrists to the subject, who has to be specialized in such type of mental health.

In an embodiment, the AI module associates a specific healthcare provider to the subject by selecting an appropriate healthcare provider according to the subject's disease and by scheduling an appointment between the subject and the healthcare provider. In an embodiment, the system is configured to send a notification with the date and time of the schedule appointment to the subject, a family member of the subject and/or a contact person of the subject. In an embodiment, the system is configured to send the notification via a telephone call, a videoconference, a SMS, a mobile application message or a web application message.

Advantageously, the AI module automatically matches subjects with healthcare providers (such as specialized doctors, nurses, caregivers, therapists, counsellors...), improving the likelihood of a strong therapeutic alliance.

In an embodiment, additional information is taken into account for creating the tailored treatment plan and/or the healthcare provider association, for example, the medical history of the subject (including laboratory results, imaging studies and/or genetic markers), the family history of the subject and/or the lifestyle factors of the subject.

In a particular embodiment, the AI module is further configured for:

- receiving at least one text and/or voice input from the subject;
- estimating the probability of the subject to be following a specific emotional trend as follows:

$$P(A|B) = \frac{P(B|A) \cdot P(A)}{P(B)}$$

wherein:

- ◦ B is the text and/or voice input from the subject;
- ◦ A is the emotional trend;
- ◦ $P(A)$ is the prior probability that the subject is following the emotional trend A;
- ◦ $P(B)$ is the prior probability of finding at least one predefined word in the text and/or voice input from the subject;
- ◦ $P(B|A)$ is the conditional probability of finding the at least one predefined word in the text and/or voice input from the subject if the subject is following the emotional trend A; and
- ◦ $P(A|B)$ is the conditional probability that the subject is following the emotional trend A if the at least one predefined word is found in the text and/or voice input from the subject; and

- comparing the estimated probability of the subject to be following a specific emotional trend with a predefined range of probabilities;
- detecting a health crisis if the estimated probability is within the predefined range; and
- emitting an alarm if a health crisis is detected.

According to this embodiment, the AI module of the system of the invention is configured to analyze text and/or voice input from the subject in order to obtain the probability of the subject to being following a specific emotional trend (for example, if the subject is experiencing anxiety, sadness, depression, happiness, anger, stress or distress), and to detect if a health crisis is going to take place (for example, if the patient is having or is prone to have a suicidal ideation). A health crisis is detected if the estimated probability of the subject to be following a specific emotional trend is within a predefined range of probabilities and, in case the health crisis is detected, an alarm is emitted by the AI module, for example, to request the emergency services to intervene.

In an embodiment, the range of probabilities is defined by a healthcare provider according to a standard and the system is configured to store such range of probailities, for example, in an internal database. In an embodiment, the range of probabilities is modified by the healthcare provider according to a disease of the subject and/or according to the symptoms of the subject and/or according to the health and/or personal parameters of the subject.

When an alarm is emitted by the system, the emergency services and/or healthcare providers can intervene proactively by reaching out to subjects, offering support, counseling, or adjusting treatment plans as needed. This not only enables timely interventions but also reduces emergency room visits.

In an embodiment, the at least one predefined word is at least one keyword, phrase and/or linguistic pattern.

In an embodiment, the AI module is configured to execute Natural Language Processing (NLP) algorithms to identify keywords, phrases and/or linguistic patterns in the text and/or voice input, wherein the keywords, phrases and/or linguistic patterns are indicative of different emotional states, such as happiness, sadness, anxiety, depression, anger, or stress. With this information, P(A) is estimated as the relative frequency of occurrence of the event "finding at least one predefined word in the at least one text and/or voice input" received from the subject.

In an embodiment, the AI module is trained for estimating the probability of the subject to be following a specific emotional trend, wherein the training data set comprises a plurality of text and/or voice inputs. In an embodiment, P(B) is determined as the relative frequency of occurrence of the event "finding at least one predefined word in the text and/or voice inputs", wherein the text and/or voice inputs considered are those included in the training dataset. In an embodiment, P(B|A) is determined as the relative frequency of occurrence of the event "finding at least one predefined word in the text and/or voice inputs", wherein the text and/or voice inputs considered are those included in the subset of the training dataset that correspond to subjects following a specific emotional trend A.

In an embodiment, the AI module is configured to execute at least one Natural Language Processing (NLP) algorithm to determine the occurrence of finding the at least one word in the text and/or voice inputs from the training dataset and/or received from the subject.

In an embodiment, text and/or voice inputs are collected from social media posts of the subject, and/or from online activity of the subject, and/or from recorded conversations with family or friends of the subject, and/or from recorded telemedicine sessions with healthcare providers of the subject. Text and/or voice input can also be collected directly from the subject reports, that is, the subject can provide the system with information about their emotional state, and/or symptoms, and/or stressors, and/or mood changes, and/or feelings, and/or experiences.

In an embodiment, the system comprises AI-driven chatbots or virtual mental health assistants that can contact the subject in case a health crisis is detected. Such AI-driven chatbots or virtual mental health assistants are configured to guide the subjects toward appropriate resources such as coping strategies, crisis hotlines or connecting the subject to mental health professionals. For example, in case the AI module detects that the subject is going to have a panic attack, the AI-driven chatbot or the virtual mental health assistant can provide the subject with anti-anxiety techniques while looking for a healthcare provider who can contact the subject.

In a particular embodiment, the AI module is configured for:

- receiving a medical history of a subject;
- estimating the probability of the subject to be following a specific emotional trend as follows:

$$P(A|C) = \frac{P(C|A) \cdot P(A)}{P(C)}$$

wherein:

- ◦ C is the medical history of the subject;
- ◦ A is the emotional trend;
- ◦ $P(A)$ is the prior probability that the subject is following the emotional trend A;
- ◦ $P(C)$ is the prior probability of finding at least one predefined data in the medical history of the subject;
- ◦ $P(C|A)$ is the conditional probability of finding the at least one predefined data in the medical history of the subject if the subject is following the emotional trend A; and
- ◦ $P(A|C)$ is the conditional probability that the subject is following the emotional trend A if the at least one predefined data is found in the medical history of the subject; and

- comparing the estimated probability of the subject to be following a specific emotional trend with a predefined range of probabilities;
- detecting a health crisis if the estimated probability is within the predefined range; and
- emitting an alarm if a health crisis is detected.

According to this embodiment, the AI module of the system of the invention also analyzes the medical history of the subject in order to obtain the probability of the subject of being following a specific emotional trend (for example, if the subject has been experiencing episodes of distress lately), and to detect if a health crisis is going to take place (for example, if the patient is having or is prone to have a suicidal ideation). A health crisis is detected if the estimated probability of the subject to be following a specific emotional trend is within a predefined range of probabilities and, in case the health crisis is detected, an alarm is emitted by the AI module, for example, to request the emergency services to intervene.

In an embodiment, the range of probabilities is defined by a healthcare provider according to a standard and the system is configured to store such range of values, for example, in an internal database. In an embodiment, the range of probabilities is modified by the healthcare provider according to a disease of the subject and/or according to the symptoms of the subject and/or according to the health and/or personal parameters of the subject.

When an alarm is emitted by the system, healthcare providers can intervene proactively by reaching out to subjects, offering support, counseling, and/or adjusting treatment plans as needed. This not only enables timely interventions but also it reduces emergency room visits.

In an embodiment, medical history comprises the personal parameters of the subject, and/or the health parameters of the subject measured over time, and/or standardized cognitive and behavioral reports generated by caregivers, family members and/or healthcare providers, and/or disease diagnose reports made by the healthcare providers, and/or information provided by the subject to the system over time, including at least one of his/her emotional state, his/her symptoms, his/her stressors, his/her mood, his/her feelings, and his/her experiences.

In an embodiment, the AI module is configured to execute Natural Language Processing (NLP) algorithms to identify data (keywords, phrases and/or linguistic patterns) in the medical history of the subject, wherein the data are indicative of different emotional states, such as happiness, sadness, anxiety, depression, anger, or stress. With this information, P(A) is estimated as the relative frequency of occurrence of the event "finding at least one predefined data in the medical history of the subject".

In an embodiment, the AI module is trained for estimating the probability of the subject to be following a specific emotional trend, wherein the training data set comprises a plurality of medical histories of subjects. In an embodiment, P(C) is determined as the relative frequency of occurrence of the event "finding at least one predefined data in the medical history of the subject", wherein the medical histories considered are those included in the training dataset. In an embodiment, P(C|A) is determined as the relative frequency of occurrence of the event "finding at least one predefined data in the medical history of the subject", wherein the medical histories considered are those included in the subset of the training dataset that correspond to subjects following a specific emotional trend A.

In an embodiment, the AI module is configured to execute at least one Natural Language Processing (NLP) algorithm to determine the occurrence of finding the at least one data in the medical histories from the training dataset and/or received from the subject.

Throughout all this document, "cognitive, behavioral and disease diagnose reports" will be understood as reports generated by caregivers, family members and/or healthcare providers that summarize the subject's health status, his/her progress, his/her test results, his/her medical imaging studies, his/her genetic tests, his/her medication and any significant change in his/her health over time.

In an embodiment, the system comprises AI-driven chatbots or virtual mental health assistants that can contact the subject in case a health crisis is detected. Such AI-driven chatbots or virtual mental health assistants are configured to guide the subjects toward appropriate resources such as coping strategies, crisis hotlines or connecting the subject to mental health professionals. For example, in case the AI module detects that the subject is going to have a panic attack, the AI-driven chatbot or the virtual mental health assistant can provide the subject with anti-anxiety techniques while looking for a healthcare provider who can contact the subject.

In a particular embodiment, the AI module is further configured for:

- receiving at least one voice input from the subject;
- estimating the probability of the subject to be following a specific emotional trend as follows:

$$P(A|D) = \frac{P(D|A) \cdot P(A)}{P(D)}$$

wherein:

  ∘ D is the voice input from the subject;
  ∘ A is the emotional trend;
  ∘ $P(A)$ is the prior probability that the subject is following the emotional trend A;
  ∘ $P(D)$ is the prior probability of finding at least one predefined speech characteristic in the voice input from the subject;
  ∘ $P(D|A)$ is the conditional probability of finding the at least one predefined speech characteristic in the voice input from the subject if the subject is following the emotional trend A; and
  ∘ $P(A|D)$ is the conditional probability that the subject is following the emotional trend A if the at least one predefined speech characteristic is found in the voice input from the subject; and

- comparing the estimated probability of the subject to be following a specific emotional trend with a predefined range of probabilities;
- detecting a health crisis if the estimated probability is within the predefined range; and
- emitting an alarm if a health crisis is detected.

In an embodiment, the speech characteristic is one or more from a tune characteristic, a tone characteristic, a pitch characteristic and a voice characteristic. The speech characteristic may include specific values of one or more or tune, tone, voice and/or pitch characteristics as well as patterns of tune, tone, voice and/or pitch characteristics.

In an embodiment, the AI module is configured to execute speech recognition and voice processing algorithms to identify speech characteristics (e.g. changes in speech patterns, tone or pitch) in the voice input of the subject, wherein the speech characteristic is indicative of different emotional states, such as happiness, sadness, anxiety, depression, anger, or stress. With this information, P(A) is estimated as the relative frequency of occurrence of the event "finding at least one predefined speech characteristic in the voice input of the subject".

In an embodiment, the AI module is trained for estimating the probability of the subject to be following a specific emotional trend, wherein the training data set comprises a plurality of voice inputs of subjects. In an embodiment, P(D) is determined as the relative frequency of occurrence of the event "finding at least one predefined speech characteristic in the voice input of the subject", wherein the voice inputs considered are those included in the training dataset. In an embodiment, P(D|A) is determined as the relative frequency of occurrence of the event "finding at least one predefined speech characteristic in the voice input of the subject", wherein the voice inputs considered are those included in the subset of the training dataset that correspond to subjects following a specific emotional trend A.

In a particular embodiment, the system comprises at least one wearable device with at least one sensor configured for:

- monitoring vital signs of the subject; and/or
- monitoring activity levels of the subject; and/or
- monitoring sleep patterns of the subject; and/or
- monitoring the heart rate of the subject; and/or
- monitoring the position of the subject; and/or
- measuring the blood pressure of the subject; and/or
- measuring the SpO2 of the subject; and/or
- measuring the temperature of the subject; and/or
- measuring the level of glucose in blood of the subject.

The system of this embodiment comprises one or more wearable devices configured for measuring and/or monitoring one or more health parameters punctually or continuously over time. Throughout all this document, "wearable device" will be understood as a device that is directly attached to human skin or clothes and that comprises at least one sensor for gathering biological signals. Examples of wearable devices are fitness trackers (smartwatch, wristband), smart glasses or smart t-shirt. Examples of sensors comprised in the wearable devices are biosensors, ECG monitors, blood pressure cuffs, and glucose monitors.

Throughout all this document, "SpO2" will be understood as the level of oxygen in blood.

In this embodiment, one or more physiological and behavioral parameters, which are specific types of health parameters, are measured or monitored by the sensors of wearable devices. In other embodiments, other health parameters can be measured or monitored by wearable devices as long as they are suitable in the context of this invention.

The health parameters provided by the wearable devices allow the AI module to make informed decisions in real time about the health of a subject, letting the healthcare providers to timely act in case it is needed.

In an embodiment, the wearable device that monitors the position of the subject is based on one of the following technologies: GPS, Beacon, Bluetooth or geofencing.

In an embodiment, the wearable device that monitors the heart rate of the subject is configured to track the subject's heart rate trend, the subject's heart rate when the subject is resting and/or variations in the subject's heart rate when the subject is carrying out different activities.

In an embodiment, the wearable device that monitors the activity levels of the subject is configured to track the steps taken by the subject over a period of time, the distance travelled by the subject over a period of time and/or the calorie expenditure of the subject over a period of time.

In an embodiment, the wearable device that monitors the sleep patterns of the subject is configured to track the sleep duration of the subject, the quality of the subject's sleep and/or the disruptions during the subject's sleep.

In an embodiment, the subject is registered in the system and the wearable device is configured to be paired with a subject's account using a subject's device, for example, a smartphone, a smartwatch or a tablet.

In an embodiment, the system is configured to check if the wearable device is placed correctly on the subject's body to obtain accurate data. If not, the subject is asked to replace the wearable device on his/her body.

In an embodiment, the wearable device is configured to transmit the health parameters that it measures in real-time or at regular intervals to the processor of the system. In an embodiment, the transmission is performed through a mobile application or a cloud-based platform.

In an embodiment, the system is configured to protect the health parameter measurements transmitted by the wearable device with security measures during the transmission and storage processes.

In an embodiment, the alarm emitted by the system comprises:

- a medical report to be sent to a healthcare provider; and/or
- an emergency call to the emergency services; and/or
- a notification, such as a notification to a healthcare provider, to the emergency services, to the subject and/or to a contact person.

As mentioned before, the system of the invention can emit alarms related with a subject's health; for example, if the system detects that the subject is developing a disease, if the system has detected a subject's mental health crisis (e.g. a suicidal ideation or a panic attack), if the system detects that a subject has fallen down or has fainted or if the system detects that a subject is out of predefined area.

The severity of the alarm is quite variable. In an embodiment, depending on the urgency, different types of alarms are issued by the system.

In an embodiment, when the alarm is urgent (for example, if a suicidal ideation or a fainting is detected), the system is configured to perform automatically an emergency call to the emergency services. When the alarm is not urgent, the system is configured to perform a call to a contact person (for example, a caregiver or a family member) informing such contact person about the health status of the subject (for example, in case a specific severity degree of a disease is detected). For both types of alarms, the system may be also configured to generate reports related with the health status of the patient automatically and to transmit such reports to the healthcare provider/s of the subject.

Advantageously, by prioritizing alarms based on severity and urgency, healthcare resources can be allocated more efficiently, improving response times and subject care.

Throughout all this document, "emergency call" will be understood as a phone call to the emergency services. Throughout all this document, "notification" will be understood as a telephone call, a mobile push notification or a message (including mobile SMS, emails, mobile application notifications or web notifications). Throughout all this document, "contact person" will be understood as any family member, caregiver, healthcare provider or any other person that the subject has decided to be contacted when certain alarms are emitted.

In an embodiment, when an alarm is emitted, the system is configured to automatically initiate emergency calls to emergency services. If there is no response to the initial emergency call, the system is further configured to send notifications to the contact person/s or alternate new emergency calls with notifications to the contact person/s.

In an embodiment, the system comprises means for tracking responses to alarms and follow-up actions.

In an embodiment, the system comprises communication means that are configured to allow the caregiver, family member or healthcare provider to communicate, at any time or particularly after an alarm emission, with the subject through a text messaging, audioconference or videoconference. Examples of communication means are mobile phones, tablets or personal computers.

In an embodiment, the communication means are further configured to allow communication among the caregiver, and/or family member and/or the healthcare provider to share updates and concerns.

In an embodiment, the system comprises customization means that are configured to allow the healthcare providers to vary the alarm settings based on individual subject needs, conditions, and preferences.

In an embodiment, the plurality of disease models comprises a heart disease model generated by the AI module using one or more of the following parameters of subjects that suffer from a heart disease:

- age,
- type of chest pain experienced by the subject,
- level of blood pressure at resting mode,
- serum cholesterol,
- maximum heart rate experienced by the subject,
- angina induced by exercise, and
- ST-depression induced by exercise in comparison with the resting mode; and wherein:

  ◦ the activation function of each node of the first hidden layer is defined by the following equation:

$$f_1(x) = \max(0, x)$$

  wherein x represents a weighted sum of the inputs of each node of the first hidden layer;
  ◦ the activation function of each node of the second hidden layer is defined by the following equation:

$$f_2(x) = x \cdot \tanh(\ln(1 + e^x))$$

  wherein x represents a weighted sum of the inputs of each node of the second hidden layer; and
  ◦ the activation function of the third hidden layer is defined by the following equation:

$$f_3(x_i) = \frac{e^{x_i}}{\sum_{i=1}^{K} e^{x_i}}$$

wherein $x_i$ represents a weighted sum of the inputs of the $i$ - $th$ node of the third hidden layer and $K$ is the total number of nodes of the third hidden layer.

In an embodiment, the heart disease model is generated by the AI module using a plurality of the mentioned parameters (i.e. a plurality of parameters selected from age, type of chest pain experienced by the subject, level of blood pressure at resting mode, serum cholesterol, maximum heart rate experienced by the subject, angina induced by exercise and ST-depression induced by exercise in comparison with the resting mode). Preferably, all the mentioned parameters (i.e. age, type of chest pain experienced by the subject, level of blood pressure at resting mode, serum cholesterol, maximum heart rate experienced by the subject, angina induced by exercise, and ST-depression induced by exercise in comparison with the resting mode) are used to generate the heart disease model.

The heart disease describes a range of conditions that affect the heart, including blood vessel disease (such as coronary artery disease), irregular heartbeats (arrhythmias), heart problems from birth (congenital heart defects), disease of the heart muscle, and heart valve disease.

The heart disease model is generated by the AI module and is based on a neural network. When the neural network receives the input parameters in the input layer, it starts the forward propagation.

In the three hidden layers of the neural network, pre-activating and activating processes take place. The pre-activation function is the calculation of the weighted sum of the inputs in a hidden layer. The activation function is applied to this weighted sum, making the neural network flow non-linearly using bias.

The first activation function used for the first hidden layer is a simple math function applied to the outputs of the input layer. Its aim is to introduce non-linearity and to enable the network to learn complex patterns and relationships in the data. This function outputs the input value if it is positive, and it outputs zero if the input value is negative.

The second activation function used for the second hidden layer is a complex math function applied to the outputs of the first hidden layer. Its aim is to introduce non-linearity and to enable the network to learn complex patterns and relationships in the data.

The third activation function used for the third hidden layer is a complex math function applied to the output of the second hidden layer. Its aim is to introduce non-linearity and to enable the network to learn complex patterns and relationships in the data.

The output layer has a single node representing the probability of the subject to suffer from the heart disease, whose inputs are the outputs of the third hidden layer.

In an embodiment, the AI module is trained with 100 epochs, what gives an accuracy of more than 90% in the subsequent AI predictions.

Throughout all this document, "ST-depression" will be understood as a ST segment of an electrocardiogram, ECG, of the subject that appears depressed or flattened compared to the baseline of the ECG. This parameter indicates a myocardial ischemia, which is a condition where there is inadequate blood flow to the heart muscle of a subject. It is often associated with coronary artery disease (CAD) or other cardiac conditions.

In an embodiment, the measurement units of the parameters are:

- years for the age, and/or
- mm/HG for the level of blood pressure at resting mode, and/or
- mg/dl for the serum cholesterol.

In an embodiment, AI module determines the probability of the subject to suffer from a heart disease using one or more of the previously mentioned parameters (i.e. age, type of chest pain experienced by the subject, level of blood pressure at resting mode, serum cholesterol, maximum heart rate experienced by the subject, angina induced by exercise, and ST-depression induced by exercise in comparison with the resting mode) as inputs of the heart disease model.

In an embodiment, the AI module uses a plurality of the previously mentioned parameters (i.e. a plurality of parameters selected from age, type of chest pain experienced by the subject, level of blood pressure at resting mode, serum cholesterol, maximum heart rate experienced by the subject, angina induced by exercise and ST-depression induced by exercise in comparison with the resting mode) in the input layer. Preferably, the AI module uses all the previously mentioned parameters (i.e. age, type of chest pain experienced by the subject, level of blood pressure at resting mode, serum cholesterol, maximum heart rate experienced by the subject, angina induced by exercise and ST-depression induced by exercise in comparison with the resting mode).

In an embodiment, the plurality of disease severity models comprises a heart disease severity model generated by the AI module using the same parameters that the AI module uses for generating the heart disease model. Additionally, the activation functions of the three hidden layers of the heart disease severity model are the same as the activation functions of the heart disease model.

In an embodiment, the AI module uses a plurality of the previously mentioned parameters (i.e. a plurality of parameters selected from age, type of chest pain experienced by the subject, level of blood pressure at resting mode,

serum cholesterol, maximum heart rate experienced by the subject, angina induced by exercise and ST-depression induced by exercise in comparison with the resting mode) in the input layer of the heart disease severity model. Preferably, the AI module uses all the previously mentioned parameters (i.e. age, type of chest pain experienced by the subject, level of blood pressure at resting mode, serum cholesterol, maximum heart rate experienced by the subject, angina induced by exercise and ST-depression induced by exercise in comparison with the resting mode).

In an embodiment, the plurality of disease models comprises a dementia disease model generated by the AI module using one or more of the following parameters of subjects that suffer from a dementia disease:

- subject suffering a diabetes disease,
- subject suffering a depression disease,
- subject with APOE $\varepsilon$3 allele,
- level of oxygen in blood,
- heart rate,
- age,
- time elapsed between the subject wears a wearable device and a magnetic resonance imaging study is performed or between a magnetic resonance imaging study is performed and the subject puts on the wearable device,
- level of alcohol in blood,
- body temperature, and
- weight;

and wherein:

  ◦ the activation function of each node of the first hidden layer is defined by the following equation:

$$f_1(x) = \max(0, x)$$

wherein $x$ represents a weighted sum of the inputs of each node of the first hidden layer;

  ◦ the activation function of each node of the second hidden layer is defined by the following equation:

$$f_2(x) = x \cdot \tanh(\ln(1 + e^x))$$

wherein $x$ represents a weighted sum of the inputs of each node of the second hidden layer; and

  ◦ the activation function of the third hidden layer is defined by the following equation:

$$f_3(x) = \ln(1 + e^x)$$

wherein $x$ represents a weighted sum of the inputs of each node of the third hidden layer.

In an embodiment, the dementia disease model is generated by the AI module using a plurality of the previously mentioned parameters (i.e. a plurality of parameters selected from subject suffering a diabetes disease, subject suffering a depression disease, subject with APOE $\varepsilon$3 allele, level of oxygen in blood, heart rate, age, time elapsed between the subject wears a wearable device and a magnetic resonance imaging study is performed or between a magnetic resonance imaging study is performed and the subject puts on the wearable device, level of alcohol in blood, body temperature, and weight) as inputs of the dementia disease model. Preferably, all the mentioned parameters (i.e. subject suffering a diabetes disease, subject suffering a depression disease, subject with APOE $\varepsilon$3 allele, level of oxygen in blood, heart rate, age, time elapsed between the subject wears a wearable device and a magnetic resonance imaging study is performed or between a magnetic resonance imaging study is performed and the subject puts on the wearable device, level of alcohol in blood, body temperature, and weight) are used to generate the dementia disease model.

Dementia is not a disease itself. It's a collection of symptoms that result from a brain damage caused by different diseases, such as Alzheimer disease. Dementia is a progressive condition that affects cognitive and functional abilities. These symptoms vary according to the part of the brain that is damaged and may include:

- memory loss; and/or
- difficulty in concentrating; and/or
- finding it hard to carry out familiar daily tasks, such as getting confused over the correct change when shopping; and/or

- struggling to follow a conversation or find the right word; and/or
- being confused about time and place; and/or
- mood changes.

The dementia disease model is generated by the AI module and it is based on a neural network. When the neural network receives the input parameters in the input layer, it starts the forward propagation.

In the three hidden layers of the neural network, pre-activating and activating processes take place. The pre-activation function is the calculation of the weighted sum of the inputs in a hidden layer. The activation function is applied to this weighted sum, making the neural network flow non-linearly using bias.

The first activation function used for the first hidden layer is a simple math function applied to the outputs of the input layer. Its aim is to introduce non-linearity and to enable the network to learn complex patterns and relationships in the data. This function outputs the input value if it is positive, and it outputs zero if the input value is negative.

The second activation function used for the second hidden layer is a complex math function applied to the outputs of the first hidden layer. Its aim is to introduce non-linearity and to enable the network to learn complex patterns and relationships in the data.

The third activation function used for the third hidden layer is a complex math function applied to the output of the second hidden layer. Its aim is to introduce non-linearity and to enable the network to learn complex patterns and relationships in the data.

The output layer has a single node representing the probability of the subject to suffer from the dementia disease, whose inputs are the outputs of the third hidden layer.

In an embodiment, the AI module is trained with 100 epochs, what gives an accuracy of more than 95% in the subsequent AI predictions.

Throughout all this document, "APOE $\varepsilon$3 allele" will be understood as a specific variant of the apolipoprotein $\varepsilon$ (APOE) gene, which plays a crucial role in lipid metabolism and cholesterol transport in the body. The presence of this gene in the subject is determined with genetic tests.

In an embodiment, the measurement units of the parameters are:

- bpm for the heart rate, and/or
- years for the age, and/or
- g/dl or mg/dl for the level of alcohol in blood, and/or
- Celsius or Fahrenheit degrees for the body temperature, and/or
- kg or lbs for the weight.

In an embodiment, AI module determines the probability of the subject to suffer from a dementia disease using one or more of the previously mentioned parameters (i.e. subject suffering a diabetes disease, subject suffering a depression disease, subject with APOE $\varepsilon$3 allele, level of oxygen in blood, heart rate, age, time elapsed between the subject wears a wearable device and a magnetic resonance imaging study is performed or between a magnetic resonance imaging study is performed and the subject puts on the wearable device, level of alcohol in blood, body temperature, and weight) as inputs of the dementia disease model.

In an embodiment, the AI module uses a plurality of the previously mentioned parameters (i.e. a plurality of parameters selected from subject suffering a diabetes disease, subject suffering a depression disease, subject with APOE $\varepsilon$3 allele, level of oxygen in blood, heart rate, age, time elapsed between the subject wears a wearable device and a magnetic resonance imaging study is performed or between a magnetic resonance imaging study is performed and the subject puts on the wearable device, level of alcohol in blood, body temperature, and weight) in the input layer. Preferably, the AI module uses all the previously mentioned parameters (i.e. subject suffering a diabetes disease, subject suffering a depression disease, subject with APOE $\varepsilon$3 allele, level of oxygen in blood, heart rate, age, time elapsed between the subject wears a wearable device and a magnetic resonance imaging study is performed or between a magnetic resonance imaging study is performed and the subject puts on the wearable device, level of alcohol in blood, body temperature, and weight).

In an embodiment wherein the AI module is further trained to generate a plurality of disease severity models, the plurality of disease severity models comprises a dementia disease severity model generated by the AI module using the same parameters that the AI module uses for generating the dementia disease model. Additionally, the activation functions of the three hidden layers of the dementia disease severity model are the same as the activation functions of the dementia disease model.

In an embodiment, the AI module uses a plurality of the previously mentioned parameters (i.e. subject suffering a diabetes disease, subject suffering a depression disease, subject with APOE $\varepsilon$3 allele, level of oxygen in blood, heart rate, age, time elapsed between the subject wears a wearable device and a magnetic resonance imaging study is performed or between a magnetic resonance imaging study is performed and the subject puts on the wearable device, level of alcohol in blood, body temperature, and weight) in the input layer of the dementia disease severity model. Preferably, the AI module uses all the previously mentioned parameters (i.e. subject suffering a diabetes disease, subject suffering a depression disease, subject with APOE ε3 allele, level of oxygen in blood, heart rate, age, time elapsed between the subject wears a wearable device and a magnetic resonance imaging study is performed or between a magnetic resonance imaging study is performed and the subject puts on the wearable device, level of alcohol in blood, body temperature, and weight).

In an embodiment, the plurality of disease models comprises a schizophrenia disease model generated by the AI module using one or more of the following parameters of subjects that suffer from a schizophrenia disease:

- sleep pattern,
- steps movements pattern, and
- heart rate,

and wherein:

○ the activation function of each node of the first hidden layer is defined by the following equation:

$$f_1(x) = \max(0, x)$$

wherein $x$ represents a weighted sum of the inputs of each node of the first hidden layer;

○ the activation function of each node of the second hidden layer is defined by the following equation:

$$f_2(x) = \max(0, x)$$

wherein $x$ represents a weighted sum of the inputs of each node of the second hidden layer; and

○ the activation function of the third hidden layer is defined by the following equation:

$$f_3(x_i) = \frac{e^{x_i}}{\sum_{i=1}^{K} e^{x_i}}$$

wherein $x_i$ represents a weighted sum of the inputs of the $i$-th node of the third hidden layer and $K$ is the total number of nodes of the third hidden layer.

In an embodiment, the schizophrenia disease model is generated by the AI module using a plurality of the previously mentioned parameters (i.e. a plurality of parameters selected from sleep pattern, steps movements pattern, and heart rate). Preferably, all the mentioned parameters (i.e. sleep pattern, steps movements pattern, and heart rate) are used to generate the schizophrenia disease model.

Schizophrenia is a mental illness that affects the way a subject thinks, feels and behaves. The condition may be developed slowly. The first signs can be hard to identify as they often are developed during the teenage years.

The symptoms of schizophrenia are usually classified into:

- positive symptoms: any change in behavior or thoughts, such as hallucinations or delusions; and
- negative symptoms: where the subject appears to withdraw from the world around him/her, takes no interest in everyday social interactions, and often appears emotionless and flat.

The schizophrenia disease model is generated by the AI module and it is based on a neural network. When the neural network receives the input parameters in the input layer, it starts the forward propagation.

In the three hidden layers of the neural network, pre-activating and activating processes take place. The pre-activation function is the calculation of the weighted sum of the inputs in a hidden layer. The activation function is applied to this weighted sum, making the neural network flow non-linearly using bias.

The first activation function used for the first hidden layer is a simple math function applied to the outputs of the input layer. Its aim is to introduce non-linearity and to enable the network to learn complex patterns and relationships in the data. This function outputs the input value if it is positive, and it outputs zero if the input value is negative.

The second activation function used for the second hidden layer is a simple math function applied to the outputs of the first hidden layer. Its aim is to introduce non-linearity and to enable the network to learn complex patterns and relationships in the data. This function outputs the input value if it is positive, and it outputs zero if the input value is negative.

The third activation function used for the third hidden layer is a complex math function applied to the output of the second hidden layer. Its aim is to introduce non-linearity and to enable the network to learn complex patterns and relationships in the data.

The output layer has a single node representing the probability of the subject to suffer from the schizophrenia disease, whose inputs are the outputs of the third hidden layer.

In an embodiment, the AI module is trained with 100 epochs, what gives an accuracy of more than 90% in the subsequent AI predictions.

In an embodiment, the measurement unit for the heart rate is bpm.

In an embodiment, AI module determines the probability of the subject to suffer from a schizophrenia disease using one or more of the previously mentioned parameters i.e. (sleep pattern, steps movements pattern, and heart rate) as inputs of the schizophrenia disease model.

In an embodiment, the AI module uses a plurality of the previously mentioned parameters (i.e. sleep pattern, steps movements pattern, and heart rate) in the input layer. Preferably, the AI module uses all the previously mentioned parameters (i.e. sleep pattern, steps movements pattern, and heart rate).

In an embodiment wherein the AI module is further trained to generate a plurality of disease severity models, the plurality of disease severity models comprises a schizophrenia disease severity model generated by the AI module using the same parameters that the AI module uses for generating the schizophrenia disease model. Additionally, the activation functions of the three hidden layers of the schizophrenia disease severity model are the same as the activation functions of the schizophrenia disease model.

In an embodiment, the AI module uses a plurality of the previously mentioned parameters (i.e. sleep pattern, steps movements pattern, and heart rate) in the input layer of the schizophrenia disease severity model. Preferably, the AI module uses all the previously mentioned parameters (i.e. sleep pattern, steps movements pattern, and heart rate).

In an embodiment, the plurality of disease models comprises an Alzheimer disease model generated by the AI module using one or more of the following parameters of subjects that suffer from an Alzheimer disease:

- clinical dementia rating, that indicates the severity of dementia symptoms;
- mini mental state examination, that indicates the degree of cognitive function;
- normalized whole brain volume,
- estimated total intracranial volume,
- atlas scaling factor, and
- age,

and wherein:

○ the activation function of each node of the first hidden layer is defined by the following equation:

$$f_1(x) = \max(0, x)$$

wherein x represents a weighted sum of the inputs of each node of the first hidden layer;

○ the activation function of each node of the second hidden layer is defined by the following equation:

$$f_2(x) = \max(0, x)$$

wherein x represents a weighted sum of the inputs of each node of the second hidden layer; and

○ the activation function of the third hidden layer is defined by the following equation:

$$f_3(x_i) = \frac{e^{x_i}}{\sum_{i=1}^{K} e^{x_i}}$$

wherein $x_i$ represents a weighted sum of the inputs of the $i$-$th$ node of the third hidden layer and $K$ is the total number of nodes of the third hidden layer.

In an embodiment, the Alzheimer disease model is generated by the AI module using a plurality of the previously mentioned parameters (i.e. a plurality of parameters selected from clinical dementia rating, mini mental state examination, normalized whole brain volume, estimated total intracranial volume, atlas scaling factor, and age). Preferably, all the mentioned parameters (i.e. clinical dementia rating, mini mental state examination, normalized whole brain volume, estimated total intracranial volume, atlas scaling factor, and age) are used to generate the Alzheimer disease model.

Alzheimer is a progressive disease, where dementia symptoms gradually worsen over several years. In its early stages, memory loss is mild, but with late-stage Alzheimer disease, subjects lose the ability to carry on a conversation and respond to their environment.

The Alzheimer disease model is generated by the AI module and it is based on a neural network. When the neural network receives the input parameters in the input layer, it starts the forward propagation.

In the three hidden layers of the neural network, pre-activating and activating processes take place. The pre-activation function is the calculation of the weighted sum of the inputs in a hidden layer. The activation function is applied to this weighted sum, making the neural network flow non-linearly using bias.

The first activation function used for the first hidden layer is a simple math function applied to the outputs of the input layer. Its aim is to introduce non-linearity and to enable the network to learn complex patterns and relationships in the data. This function outputs the input value if it is positive, and it outputs zero if the input value is negative.

The second activation function used for the second hidden layer is a simple math function applied to the outputs of the first hidden layer. Its aim is to introduce non-linearity and to enable the network to learn complex patterns and relationships in the data. This function outputs the input value if it is positive, and it outputs zero if the input value is negative.

The third activation function used for the third hidden layer is a complex math function applied to the output of the second hidden layer. Its aim is to introduce non-linearity and to enable the network to learn complex patterns and relationships in the data.

The output layer has a single node representing the probability of the subject to suffer from the Alzheimer disease, whose inputs are the outputs of the third hidden layer.

In an embodiment, the AI module is trained with 100 epochs, what gives an accuracy of more than 94% in the subsequent AI predictions.

Throughout all this document, "mini mental state examination" will be understood as a parameter that represents the degree of cognitive function punctuated in a scale. Preferable, the scale ranges from 0 to 30, wherein higher scores indicates better cognitive function of the subject.

Throughout all this document, "clinical dementia rating" will be understood as a parameter that represents the severity of dementia symptoms with a predefined scale. Preferably, the scale ranges from 0 to 3, wherein higher scores indicates more severe dementia symptoms.

In an embodiment, the measurement units of the parameters are:

- cc or ml for the normalized whole brain volume, and/or
- cc or ml for the estimated total intracranial volume, and/or
- years for the age.

In an embodiment, the parameters normalized whole brain volume, the estimated total intracranial volume, and the atlas scaling factor are based on a subject's x-ray study.

In an embodiment, AI module determines the probability of the subject to suffer from an Alzheimer disease using one or more of the previously mentioned parameters (i.e. clinical dementia rating, mini mental state examination, normalized whole brain volume, estimated total intracranial volume, atlas scaling factor, and age) as inputs of the Alzheimer disease model.

In an embodiment, the AI module uses a plurality of the previously mentioned parameters (i.e. clinical dementia rating, mini mental state examination, normalized whole brain volume, estimated total intracranial volume, atlas scaling factor, and age) in the input layer. Preferably, the AI module uses all the previously mentioned parameters (i.e. clinical dementia rating, mini mental state examination, normalized whole brain volume, estimated total intracranial volume, atlas scaling factor, and age).

In an embodiment wherein the AI module is further trained to generate a plurality of disease severity models, the plurality of disease severity models comprises an Alzheimer disease severity model generated by the AI module using the same parameters that the AI module uses for generating the Alzheimer disease model. Additionally, the activation functions of the three hidden layers of the Alzheimer disease severity model are the same as the activation functions of the Alzheimer disease model.

In an embodiment, the AI module uses a plurality of the previously mentioned parameters (i.e. clinical dementia rating, mini mental state examination, normalized whole brain volume, estimated total intracranial volume, atlas scaling factor, and age) in the input layer of the Alzheimer disease severity model. Preferably, the AI module uses all the previously mentioned parameters (i.e. clinical dementia rating, mini mental state examination, normalized whole brain volume, estimated total intracranial volume, atlas scaling factor, and age).

In an embodiment, the plurality of disease models comprises an anxiety disease model and/or a depression disease model generated by the AI module using one or more of the following parameters of subjects that suffer from an anxiety disease and/or a depression disease:

- heart rate variability;
- electrodermal activity;
- respiratory rate and respiratory patterns;
- level of blood pressure;
- activity levels;
- sleep pattern;
- social interaction pattern;
- substance use or abuse history;
- self-reported symptoms of anxiety and/or depression;
- history of mental health diagnoses or treatments;

and wherein:

∘ the activation function of each node of the first hidden layer is defined by the following equation:

$$f_1(x) = \max(0, x)$$

wherein $x$ represents a weighted sum of the inputs of each node of the first hidden layer;

∘ the activation function of each node of the second hidden layer is defined by the following equation:

$$f_2(x) = \frac{1}{1 + e^{-x}}$$

wherein $x$ represents a weighted sum of the inputs of each node of the second hidden layer; and

∘ the activation function of the third hidden layer is defined by the following equation:

$$f_3(x) = \frac{e^x - e^{-x}}{e^x + e^{-x}}$$

wherein $x$ represents a weighted sum of the inputs of each node of the third hidden layer.

In an embodiment, the anxiety disease and/or the depression disease models is/are generated by AI module using a plurality of the previously mentioned parameters (i.e. a plurality of parameters selected from heart rate variability, electrodermal activity, respiratory rate and respiratory patterns, level of blood pressure, activity levels, sleep pattern, social interaction pattern, substance use or abuse history, self-reported symptoms of anxiety and/or depression, and history of mental health diagnoses or treatments). Preferably, all the mentioned parameters (i.e. heart rate variability, electrodermal activity, respiratory rate and respiratory patterns, level of blood pressure, activity levels, sleep pattern, social interaction pattern, substance use or abuse history, self-reported symptoms of anxiety and/or depression, and history of mental health diagnoses or treatments) are used to generate the anxiety disease and/or the depression disease models.

Depression (also known as major depression, major depressive disorder, or clinical depression) is a common but serious mood disorder. It causes severe symptoms that affect how a subject feels, thinks, and handles daily activities, such as sleeping, eating, or working.

Anxiety disorders involve more than temporary worry or fear. For people with an anxiety disorder, the anxiety does not go away and can get worse over time. The symptoms can interfere with daily activities such as job performance, schoolwork, and relationships.

In an embodiment, the measurement units of the parameters are:

- bpm for the heart rate variability, and/or
- breaths per minute for the respiratory rate, and/or
- mm/HG for the level of blood pressure.

The depression disease model and/or the anxiety disease model is generated by the AI module and it is based on a neural network. When the neural network receives the input parameters in the input layer, it starts the forward propagation.

In the three hidden layers of the neural network, pre-activating and activating processes take place. The pre-activation function is the calculation of the weighted sum of the inputs in a hidden layer. The activation function is applied to this weighted sum, making the neural network flow non-linearly using bias.

The first activation function used for the first hidden layer is a simple math function applied to the outputs of the input layer. Its aim is to introduce non-linearity and to enable the network to learn complex patterns and relationships in the data. This function outputs the input value if it is positive, and it outputs zero if the input value is negative.

The second activation function used for the second hidden layer is a complex math function applied to the outputs of the first hidden layer. Its aim is to introduce non-linearity and to enable the network to learn complex patterns and relationships in the data.

The third activation function used for the third hidden layer is a complex math function applied to the output of the second hidden layer. Its aim is to introduce non-linearity and to enable the network to learn complex patterns and relationships in the data.

The output layer has a single node representing the probability of the subject to suffer from the depression or anxiety disease, whose inputs are the outputs of the third hidden layer.

In an embodiment, the AI module is trained with 100 epochs, what gives an accuracy of more than 95% in the subsequent AI predictions.

Throughout all this document, "social interaction pattern" will be understood as a parameter that indicates the manner in which the subject engages with other people in social settings, both in person and through digital communication channels.

In an embodiment, the social interaction pattern comprises the frequency of interaction, that is, how often the subject engages in social interactions with other people. In an embodiment, it ranges from frequent interactions with a large network of contacts to more sporadic or infrequent interactions with a smaller group of close associates.

In an embodiment, the social interaction pattern comprises the types of interactions, such as face-to-face conversations, phone calls, text messages, emails, social media interactions, and participation in group activities or events.

In an embodiment, the social interaction pattern comprises the communication styles, such as assertive, passive, or aggressive communication styles. In an embodiment, communication styles vary based on cultural norms, personal preferences, and the nature of the relationship.

In an embodiment, the social interaction pattern comprises the quality of the relationships. In an embodiment, the quality of the relationship is determined taking into account at least one of the following factors: trust, mutual respect, empathy, and supportiveness within relationships.

In an embodiment, the social interaction pattern comprises the subject's social network structure, which is determined based on at least one of the following parameters: the size, the density, and the diversity of connections. For example, some subjects may have broad and diverse social networks, while others may have smaller, more tightly knit networks.

In an embodiment, the social interaction pattern comprises the subject's digital interaction patterns, which is determined according to at least one of the following parameters: the social media engagement, the online messaging, the video calls, and the participation in virtual communities or forums.

Throughout all this document, "activity level" will be understood as the amount and/or intensity of physical activity and/or movement that the subject engages in over a specific period. For example, some of the activities that can contribute to the activity level are exercise, walking, running, cycling, swimming, and participating in sports or recreational activities.

In an embodiment, AI module determines the probability of the subject to suffer from an anxiety disease and/or a depression disease using one or more of the previously mentioned parameters (i.e. heart rate variability, electrodermal activity, respiratory rate and respiratory patterns, level of blood pressure, activity levels, sleep pattern, social interaction pattern, substance use or abuse history, self-reported symptoms of anxiety and/or depression, and history of mental health diagnoses or treatments) as inputs of the anxiety disease and/or a depression disease models.

In an embodiment, the AI module uses a plurality of the previously mentioned parameters (i.e. heart rate variability, electrodermal activity, respiratory rate and respiratory patterns, level of blood pressure, activity levels, sleep pattern, social interaction pattern, substance use or abuse history, self-reported symptoms of anxiety and/or depression, and history of mental health diagnoses or treatments) in the input layer. Preferably, the AI module uses all the previously mentioned parameters (i.e. heart rate variability, electrodermal activity, respiratory rate and respiratory patterns, level of blood pressure, activity levels, sleep pattern, social interaction pattern, substance use or abuse history, self-reported symptoms of anxiety and/or depression, and history of mental health diagnoses or treatments).

In an embodiment wherein the AI module is further trained to generate a plurality of disease severity models, the plurality of disease severity models comprises an anxiety disease severity model and/or a depression disease severity model generated by the AI module using the same parameters that the AI module uses for generating the anxiety disease model and/or the depression disease model. Additionally, the activation functions of the three hidden layers of the anxiety disease severity model and/or the depression disease severity model are the same as the activation functions of the anxiety disease model and/or the depression disease model, respectively.

In an embodiment, the AI module uses a plurality of the previously mentioned parameters (i.e. heart rate variability, electrodermal activity, respiratory rate and respiratory patterns, level of blood pressure, activity levels, sleep pattern, social interaction pattern, substance use or abuse history, self-reported symptoms of anxiety and/or depression, and history of mental health diagnoses or treatments) in the input layer of the anxiety disease severity model and/or the depression disease severity model. Preferably, the AI module uses all the previously mentioned parameters (i.e. heart rate variability, electrodermal activity, respiratory rate and respiratory patterns, level of blood pressure, activity levels, sleep pattern, social interaction pattern, substance use or abuse history, self-reported symptoms of anxiety and/or depression, and history of mental health diagnoses or treatments).

In an embodiment, the plurality of disease models comprises an eating disorder disease model generated by the AI module using one or more of the following parameters of subjects that suffer from an eating disorder disease:

- age;
- sex;
- body mass index;
- number of binge episodes per week;
- number of purging episodes per week;
- body dissatisfaction score, that indicates the score of self-reported body dissatisfaction;
- eating disorder examination questionnaire score, that indicates the score of the eating disorder symptoms according to a standard;
- depression score, that indicates the score from a depression assessment scale;
- anxiety score, that indicates the score from an anxiety assessment scale; and
- eating disorder diagnosis;

and wherein:

○ the activation function of each node of the first hidden layer is defined by the following equation:

$$f_1(x) = \max(0, x)$$

wherein $x$ represents a weighted sum of the inputs of each node of the first hidden layer;

○ the activation function of each node of the second hidden layer is defined by the following equation:

$$f_2(x) = \frac{1}{1 + e^{-x}}$$

wherein $x$ represents a weighted sum of the inputs of each node of the second hidden layer; and

○ the activation function of the third hidden layer is defined by the following equation:

$$f_3(x) = \frac{e^x - e^{-x}}{e^x + e^{-x}}$$

wherein $x$ represents a weighted sum of the inputs of each node of the third hidden layer.

In an embodiment, the eating disorder disease model is generated by the AI module using a plurality of the previously mentioned parameters (i.e. a plurality of parameters selected from age, sex, body mass index, number of binge episodes per week, number of purging episodes per week, body dissatisfaction score, eating disorder examination questionnaire score, depression score, anxiety score, and eating disorder diagnosis). Preferably, all the mentioned parameters (i.e. age, sex, body mass index, number of binge episodes per week, number of purging episodes per week, body dissatisfaction score, eating disorder examination questionnaire score, depression score, anxiety score, and eating disorder diagnosis) are used to generate the eating disorder disease model.

An eating disorder is a mental health condition where a subject uses the control of food to cope with feelings and other situations.

The eating disorder disease model is generated by the AI module and is based on a neural network. When the neural network receives the input parameters in the input layer, it starts the forward propagation.

In the three hidden layers of the neural network, pre-activating and activating processes take place. The pre-activation function is the calculation of the weighted sum of the inputs in a hidden layer. The activation function is applied to this weighted sum, making the neural network flow non-linearly using bias.

The first activation function used for the first hidden layer is a simple math function applied to the outputs of the input layer. Its aim is to introduce non-linearity and to enable the network to learn complex patterns and relationships in the data. This function outputs the input value if it is positive, and it outputs zero if the input value is negative.

The second activation function used for the second hidden layer is a complex math function applied to the outputs of the first hidden layer. Its aim is to introduce non-linearity and to enable the network to learn complex patterns and relationships in the data.

The third activation function used for the third hidden layer is a complex math function applied to the output of the second hidden layer. Its aim is to introduce non-linearity and to enable the network to learn complex patterns and relationships in the data.

The output layer has a single node representing the probability of the subject to suffer from the eating disorder disease, whose inputs are the outputs of the third hidden layer.

In an embodiment, the AI module is trained with 100 epochs, what gives an accuracy of more than 96% in the subsequent AI predictions.

In an embodiment, the measurement units of the parameters are:

- years for the age, and/or
- $kg/m^2$ for the body mass index.

Throughout all this document, "body dissatisfaction score" will be understood as a score obtained from a self-reported questionnaire assessing his/her body dissatisfaction.

Throughout all this document, "eating disorder examination questionnaire score" will be understood as a score obtained from the Eating Disorder Examination Questionnaire, a standardized assessment scale for eating disorder symptoms.

Throughout all this document, "depression score" will be understood as a score reported by the subject according to a depression assessment scale; for example, the Beck Depression Inventory scale.

Throughout all this document, "anxiety score" will be understood as a score reported by the subject according to an anxiety assessment scale; for example, Generalized Anxiety Disorder 7-item scale.

Throughout all this document, "eating disorder diagnose" will be understood as a parameter that indicates if the subject has mental health problems and/or compulsivity and/or anxiety.

In an embodiment, AI module determines the probability of the subject to suffer from an eating disorder disease using the one or more of the previously mentioned parameters (i.e. age, sex, body mass index, number of binge episodes per week, number of purging episodes per week, body dissatisfaction score, eating disorder examination questionnaire score, depression score, anxiety score, and eating disorder diagnosis) as inputs of the eating disorder disease model.

In an embodiment, the AI module uses a plurality of the previously mentioned parameters (i.e. age, sex, body mass index, number of binge episodes per week, number of purging episodes per week, body dissatisfaction score, eating disorder examination questionnaire score, depression score, anxiety score, and eating disorder diagnosis) in the input layer. Preferably, the AI module uses all the previously mentioned parameters (i.e. age, sex, body mass index, number of binge episodes per week, number of purging episodes per week, body dissatisfaction score, eating disorder examination questionnaire score, depression score, anxiety score, and eating disorder diagnosis).

In an embodiment wherein the AI module is further trained to generate a plurality of disease severity models, the plurality of disease severity models comprises an eating disorder disease severity model generated by the AI module using the same parameters that the AI module uses for generating the eating disorder disease model. Additionally, the activation functions of the three hidden layers of the eating disorder disease severity model are the same as activation functions of the eating disorder disease model.

In an embodiment, the AI module uses a plurality of the previously mentioned parameters (i.e. age, sex, body mass index, number of binge episodes per week, number of purging episodes per week, body dissatisfaction score, eating disorder examination questionnaire score, depression score, anxiety score, and eating disorder diagnosis) in the input layer of the eating disorder disease severity model. Preferably, the AI module uses all the previously mentioned parameters (i.e. age, sex, body mass index, number of binge episodes per week, number of purging episodes per week, body dissatisfaction score, eating disorder examination questionnaire score, depression score, anxiety score, and eating disorder diagnosis).

All the features described in this specification (including the claims, description and drawings) can be combined in any combination, with the exception of combinations of such mutually exclusive features.

## DESCRIPTION OF THE DRAWINGS

These and other characteristics and advantages of the invention will become clearly understood in view of the detailed description of the invention which becomes apparent from a preferred embodiment of the invention, given just as an example and not being limited thereto, with reference to the drawings.

Figure 1a        This figure shows the system according to an embodiment of the invention.
Figure 1b        This figure shows a subject wearing several wearable devices comprising sensors.
Figure 2         This figure shows the neural network on which the disease models are based.
Figures 3a-3e    These figures show a decision tree used to make a selection of health and personal parameters before the training of the AI module (Figure 3a) and several examples of selected parameters for different disease models (Figures 3b-3e).
Figures 4a-4b    These figures show an example of table created with medical database data for training the AI module.

## DETAILED DESCRIPTION OF THE INVENTION

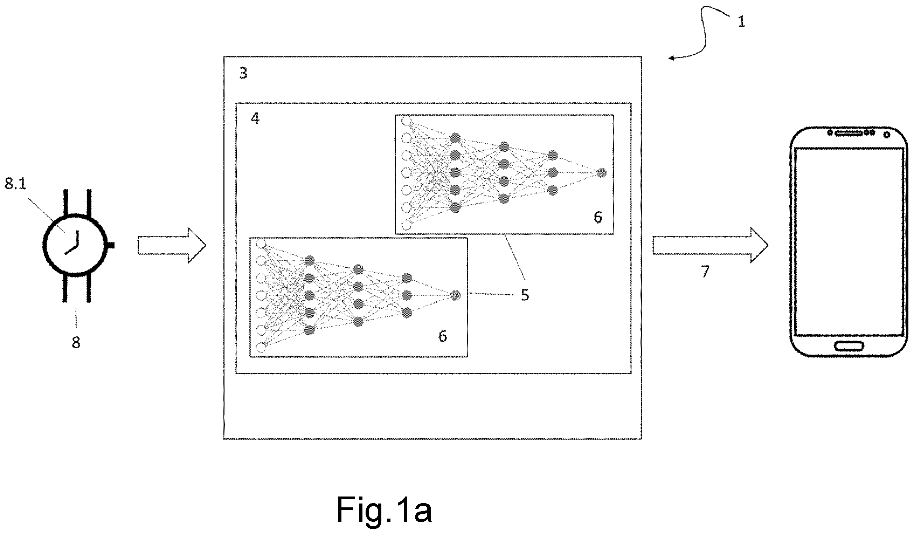

Figure 1a schematically shows a system (1) for emitting alarms related to the health of a subject (2) according to an embodiment of the invention, the system (1) comprising a processor (3) with an Artificial Intelligence (AI) module (4).
The processor (3) is configured to:

- receive at least one health parameter of a subject, wherein one or more of the at least one health parameter is measured by at least one sensor (8.1), and
- receive at least one personal parameter of the subject.

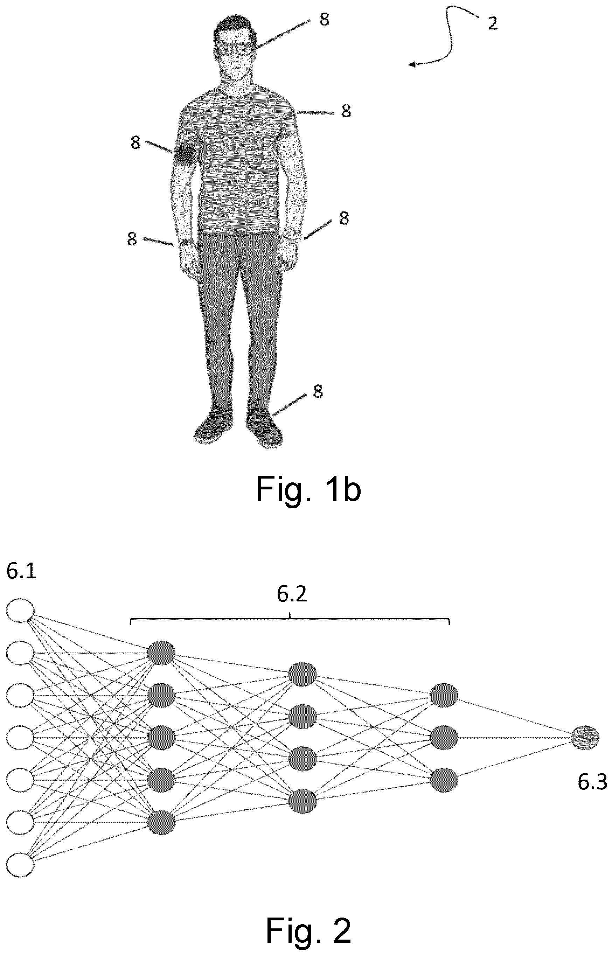

Examples of health parameters are the blood pressure of the subject (2), the level of glucose in blood of the subject (2), the sleep pattern of the subject (2) or the body temperature of the subject (2). Examples of personal parameters are the sex or the age of the subject (2).
In an embodiment, the at least one sensor (8.1) is part of the system (1), particularly being inside a smartwatch (8) - a specific type of wearable device (8). In other embodiments, the sensors (8.1) are not part of the system (1). In other embodiments, the sensors (8.1) are not inside a wearable device (8).
In this embodiment, the AI module (4) is trained to generate a plurality of disease models (5), the training being carried out with at least one health parameter and/or at least one personal parameter of subjects (2) that suffer from at least one of the diseases of the disease models (5) and at least one health parameter and/or at least one personal parameter of subjects (2) that do not suffer from said disease. Such training data is taken, for example, from medical databases.
Each disease model (5) is based on a neural network (6). The structure of the neural networks of the disease models (5) is schematically shown in Figure 2.
Each neural network (6) comprises:

○ an input layer (6.1) comprising N nodes, N being the number of health parameters and personal parameters used for the training of the disease model (5);
○ three hidden layers (6.2), each one comprising an activation function and a plurality of nodes configured to receive a plurality of inputs; and
○ an output layer (6.3) with a single node representing the probability of the subject to suffer from the disease.

The output of each node of each hidden layer (6.2) of the neural network (6) is the result of applying its corresponding activation function to a weighted sum of its corresponding inputs.
The AI module (4) is configured to carry out the following steps:

a) using the at least one health parameter and/or the at least one personal parameter as input for one or more of the disease models (5);
b) determining the probability of the subject to suffer from a disease; and
c) emitting an alarm (7) if the probability exceeds a determined probability value.

In Figure 1a the emission of the alarm (7) has been schematically depicted as the transmission of a notification to a device, such as a mobile phone or a tablet. However, this is only for illustrative purposes, since different types of alarms (7) may be issued.

In Figure 1b, a subject (2) is shown wearing several wearable devices (8) with sensors (8.1). In an embodiment, such wearable devices (8) and sensors (8.1) are not part of the system (1). In other embodiment, at least one of the wearable devices (8) with sensors (8.1) is part of the system (1).

In this particular example of Figure 1b, the wearable devices (8) are:

- smart glasses with GPS, vision check sensor, heart-rate sensor and blood pressure sensor;
- a smart t-shirt with GPS, temperature sensor, metabolites sensor, heart-rate sensor and blood pressure sensor;
- a microneedle patch with tracking sensor, glucose sensor, heart-rate sensor, blood pressure sensor, SpO2 sensor and temperature sensor;
- a wristband with tracking sensor, glucose sensor, heart-rate sensor, blood pressure sensor, SpO2 sensor, metabolites sensor and temperature sensor;
- a smartwatch with GPS, glucose sensor, heart-rate sensor, blood pressure sensor, SpO2 sensor, metabolites sensor and temperature sensor;
- smart shoes with tracking sensor, heart-rate sensor, blood pressure sensor, sensor to detect neurological conditions, motion sensor, sweat level sensor and Parkinson monitor sensor.

In this example, some of the wearable (8) devices comprise GPS or tracking sensors (8.1). In an embodiment, such GPS and tracking sensors (8.1) are configured for tracking the location of the subject (2) and the AI module (4) is further configured to emit an alarm (7) if the subject (2) is out of a predefined area, for example, his/her house or a hospital room.

In an embodiment, the smartwatch (8) and/or the wristband (8) and/or the smart t-shirt (8) and/or the smartshoes (8) further comprises a three axis gyroscope (8.1) and a three axis accelerometer (8.1) and the AI module (4) is further configured for

- detecting if the subject (2) has fainted or has fallen down by analyzing the angular velocity measured by the three axis gyroscope (8.1) and the acceleration measured by the three axis accelerometer (8.1), and
- emitting an alarm (7) in case the subject (2) has fainted or has fallen down.

Although a plurality of wearable devices each comprising a plurality of sensors is shown in the embodiment of Figure 1b, in other embodiments a different type and/or number or sensors may be used. Also, said sensors may be included or not in wearable devices.

In an embodiment, the system (1) comprises at least one wearable device (8) with at least one sensor (8.1) configured for:

- monitoring vital signs of the subject (2); and/or
- monitoring activity levels of the subject(2); and/or
- monitoring sleep patterns of the subject (2); and/or
- monitoring the heart rate of the subject (2); and/or
- monitoring the position of the subject (2); and/or
- measuring the blood pressure of the subject (2); and/or
- measuring the SpO2 of the subject (2); and/or
- measuring the temperature of the subject (2); and/or
- measuring the level of glucose in blood of the subject (2).

In an embodiment, the subject (2) is registered in the system (1) and the wearable device (8) is paired with a subject's account using a subject's device, for example, a smartphone, a smartwatch or a tablet.

In an embodiment, the system (1) is configured to check if the wearable device (8) is placed correctly on the subject's (2) body to obtain accurate data. If not, the subject (2) is asked to replace the wearable device (8) on his/her body.

In an embodiment, the wearable device (8) is configured to transmit the health parameters that it measures in real-time or at regular intervals to the processor (3) of the system (1). In an embodiment, the transmission is performed through a mobile application or a cloud-based platform.

In an embodiment, the system (1) is configured to protect the health parameter measurements transmitted by the wearable device (8) with security measures during the transmission and storage processes.

In an embodiment, the AI module (4) is further configured for:

- recommending a personalized treatment plan according to the probability of the subject (2) to suffer from a disease;

and/or

- associating a subject (2) with at least one specific healthcare provider according to the probability of the subject (2) to suffer from a disease.

In an embodiment, the system (1) comprises a database with a plurality of treatments intended for treating a specific disease.

In an embodiment, the AI module (4) is further configured for providing explanations and justifications for the recommended treatment plans, highlighting the underlying rationale, evidence-based guidelines, and factors influencing the decision-making process.

In an embodiment, the system (1) is configured to receive reports (e.g. from the healthcare providers), wherein the reports indicate how the subject (2) responses to previously prescribed treatments, for example, to previous medication plans. The reports can also include the symptoms that the subject (2) manifests when the subject (2) followed the prescribed treatment plan. The AI module (4) is further configured to select a specific treatment plan for the subject (2) taken into account the reports.

In an embodiment, the system (1) is configured to receive reports (e.g. from the healthcare providers), wherein the reports indicate how the subject (2) responses to the recommended treatment, for example, to the medication plans. The reports can also include the symptoms that the subject (2) manifests when the subject (2) is following the recommended plan. The AI module (4) is further configured to improve the treatment plans for the subject (2) taking into account the reports.

In an embodiment, the system (1) is configured to transmit data to and receive data from clinical decision support tools used by healthcare providers.

In an embodiment, the AI module (4) of the system (1) is further configured for generating collaborative treatment plans to be followed by both healthcare providers and caregivers, the collaborative treatment plans being based on the subject's (2) changing needs and responses to treatment.

In an embodiment, the system (1) is configured for feeding the AI module (4) automatically with information from new subjects (2) suffering from one or more diseases, and/or treatment outcomes, and/or medical literature updates to train the AI module. This allows to refine and improve the accuracy and effectiveness of the treatment recommendations over time.

In an embodiment, the system (1) is configured to provide resources and access to online support groups for caregivers and family members.

In an embodiment, the system (1) is configured to provide access to the subject's family members to relevant information about his/her condition and to receive updates.

In an embodiment, the AI module (4) is further configured for

- receiving at least one text and/or voice inputsfrom the subject (2);
- estimating the probability of the subject (2) to be following a specific emotional trend as follows:

$$P(A|B) = \frac{P(B|A) \cdot P(A)}{P(B)}$$

wherein:

- B is the text and/or voice input from the subject (2);
- A is the emotional trend;
- P(A) is the prior probability that the subject (2) is following the emotional trend A;
- P(B) is the prior probability of finding at least one predefined word in the text and/or voice input from the subject (2);
- P(B|A) is the conditional probability of finding the at least one predefined word in the text and/or voice input from the subject (2) if the subject (2) is following the emotional trend A; and
- P(A|B) is the conditional probability that the subject (2) is following the emotional trend A if the at least one predefined word is found in the text and/or voice input from the subject (2); and

- comparing the estimated probability of the subject to be following a specific emotional trend with a predefined range of probabilities;
- detecting a health crisis if the estimated probability is within the predefined range; and
- emitting an alarm if a health crisis is detected.

In an embodiment, the AI module (4) is configured for:

- receiving a medical history of a subject (2);
- estimating the probability of the subject (2) to be following a specific emotional trend as follows:

$$P(A|C) = \frac{P(C|A) \cdot P(A)}{P(C)}$$

wherein:

○ C is the medical history of the subject (2);
○ A is the emotional trend;
○ $P(A)$ is the prior probability that the subject (2) is following the emotional trend A;
○ $P(C)$ is the prior probability of finding at least one predefined data in the medical history of the subject (2);
○ $P(C|A)$ is the conditional probability of finding the at least one predefined data in the medical history of the subject (2) if the subject (2) is following the emotional trend A; and
○ $P(A|C)$ is the conditional probability that the subject (2) is following the emotional trend A if the at least one predefined data is found in the medical history of the subject (2); and

- comparing the estimated probability of the subject to be following a specific emotional trend with a predefined range of probabilities;
- detecting a health crisis if the estimated probability is within the predefined range; and
- emitting an alarm if a health crisis is detected.

In an embodiment, the system (1) comprises AI-driven chatbots or virtual mental health assistants that can contact the subject (2) in case a health crisis is detected.

In an embodiment, the alarm (7) comprises:

- a medical report to be sent to a healthcare provider; and/or
- an emergency call to the emergency services; and/or
- a notification to the subject and/or to a contact person.

In an embodiment, the processor (3) of the system (1) is within a server. The subject (2), and/or the healthcare providers, and/or the family members and/or the caregivers access the system (1) through mobile or web applications.

In an embodiment, the mobile or web applications have an intuitive and user-friendly interface for easy navigation.

In an embodiment, the system (1) is configured to transmit data to and receive data from other healthcare systems and electronic health records (EHRs) to facilitate seamless information exchange among healthcare providers.

In an embodiment, the system (1) comprises security measures to protect sensitive subject (2) information; for example, data encryption, access controls, and regular security audits.

In an embodiment, the subject (2) has to be registered in the mobile or web application so that a relationship is established between the specific subject (2) and his/her caregivers, healthcare providers, and family members. Besides, thanks to the registration, a specific health profile is created for this subject (2), the profile including personal information, emergency contacts and historical medical records.

In an embodiment, the methods for subject (2) authentication and authorization with the system (1) APIs are API keys, OAuth tokens, or certificates. In an embodiment, the system (1) provides with different access levels and permissions for different types of users (e.g., personal data of the subject (2), health metrics) based on the user role (subject, healthcare provider, family member or caregiver) and wearable device (8) capabilities, if any.

In an embodiment, the system (1) is configured to send through the mobile or web application medication reminders to caregivers and/or subjects (2), for example in the case of subjects with dementia or other mental diseases, to ensure that medications are taken on time and in the correct dosage.

In an embodiment, the system (1) is configured to store a record of medication schedules, dosages, and any side effects so that the healthcare providers can access it through the mobile or web application.

In an embodiment, the system (1) is configured to show schedules, dosages, and routines to healthcare providers through the mobile or web application to improve their daily assistance to the subjects.

In an embodiment, the system (1) is configured to send automated appointment reminders to caregivers and/or subjects through the mobile or web application to reduce the likelihood of missed appointments.

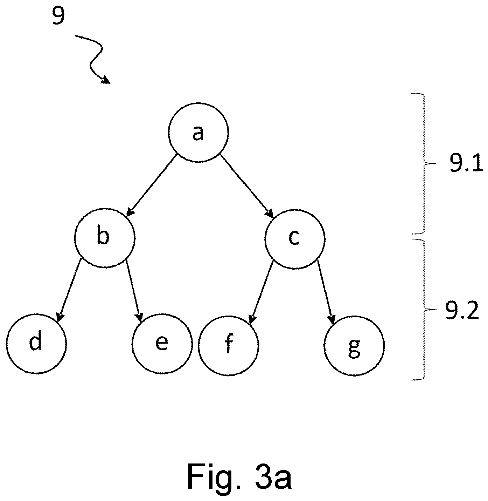

Figure 3a shows a decision tree (9) used to make a selection of health and personal parameters of each disease model (5). In particular, the processor (3) of the system (1) is configured to select the health parameters and/or the personal parameters used to train the AI module (4) for generating each disease model (5). The selection is carried out using a plurality of decision trees (9), each decision tree (9) comprising a plurality of layers (9.1, 9.2), and each layer (9.1. 9.2) comprising at least one parent node, at least one left child node and at least one right child node, and wherein the processor (3) is configured to carry out the following steps:

- using the health parameters and the personal parameters as the inputs of the plurality of decision trees (9);
- determining an importance value of each health parameter and each personal parameter for each layer (9.1, 9.2) of a decision tree (9) as the decrease in node impurity weighted by the probability of reaching the at least one parent node of the layer (9.1, 9.2); wherein

    ◦ the probability of reaching the parent node of the layer (9.1, 9.2) is determined as the number of health parameters and personal parameters that reach said parent node divided by the total number of health parameters and personal parameters used to train the AI module (4); and
    ◦ the decrease in node impurity is determined as follows:

$$Decrease\ node\ impurity = I_p - \left( \frac{N_{left}}{N_{right}} \cdot I_{left} + \frac{N_{right}}{N_{left}} \cdot I_{right} \right)$$

being:

    $N_{left}$ and $N_{right}$ the total number of health parameters and personal parameters in the at least one left and the at least one right child nodes of the layer (9.1, 9.2), respectively, and
    $I_p$, $I_{left}$ and $I_{right}$ are, respectively, the impurities of the at least one parent node, the at least one left child node and the at least one right child node; the impurities being calculated with an impurity measure, preferably a Gini impurity measure or an entropy measure;

- determining a final importance value for each health parameter and each personal parameter as the importance value of said health parameter or personal parameter calculated for the last layer (9.2) of the decision tree (9); and
- selecting the health parameters and/or personal parameters to be used to train the AI module (4) as the ones with a final importance value over a predefined threshold.

The decision tree (9) of Figure 3a comprises two layers (9.1, 9.2). Node 'a' is the parent node of the first layer (9.1), node 'b' is the left child node of the first layer (9.1) and node 'c' is the right child node of the first layer (9.1). Nodes 'b' and 'c' are the parent nodes of the second layer (9.2), nodes 'd' and 'f' are the left child nodes of the second layer (9.2) and nodes 'e' and 'g' are the right child nodes of the second layer (9.2).

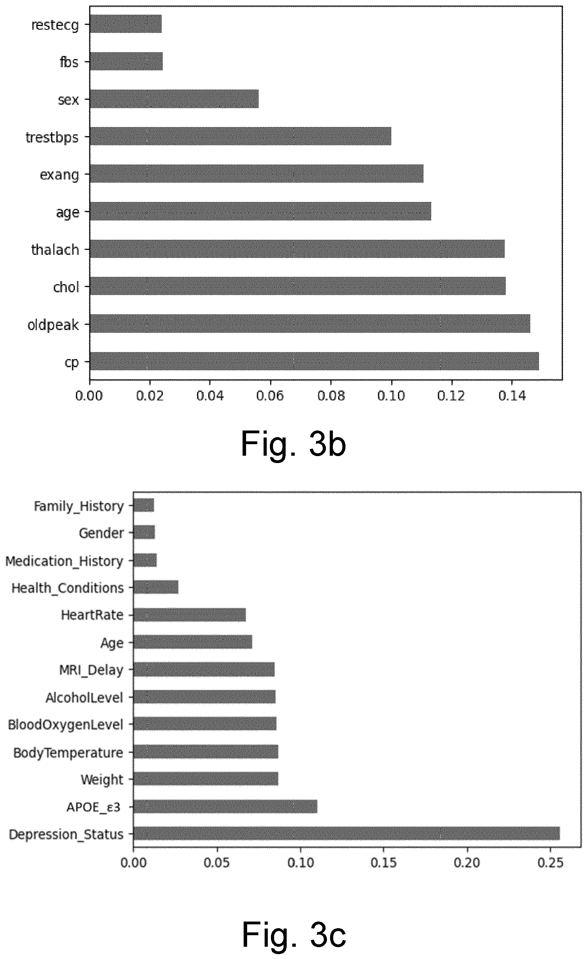

In an embodiment, the plurality of disease models (5) comprises a heart disease model (5) generated by the AI module (4). Figure 3b shows the final importance values of 10 health and personal parameters obtained with decision trees (9) for the heart disease model (5) in an example. In this case, only the parameters above a predefined threshold of 0.07 are selected to train the AI module (4) for generating such heart disease model (5):

- age,
- type of chest pain experienced by the subject (cp),
- level of blood pressure at resting mode (trestbps),
- serum cholesterol (chol),
- maximum heart rate experienced by the subject (oldpeak),
- angina induced by exercise (exang), and
- ST-depression induced by exercise in comparison with the resting mode (thalach).

The parameters that are rejected are:

- electrocardiogram measured in resting mode (restecg);
- fasting blood sugar (fbs); and
- sex.

In this embodiment, the selection of 10 health and personal parameters was performed using a random forest classifier, wherein all the possible health and personal parameters are used as input of said classifier.

For this heart disease model (5):

○ the activation function of each node of the first hidden layer (3.2) is defined by the following equation:

$$f_1(x) = \max(0, x)$$

wherein $x$ represents a weighted sum of the inputs of each node of the first hidden layer (3.2);

○ the activation function of each node of the second hidden layer (3.2) is defined by the following equation:

$$f_2(x) = x \cdot \tanh(\ln(1 + e^x))$$

wherein $x$ represents a weighted sum of the inputs of each node of the second hidden layer (3.2); and

○ the activation function of the third hidden layer (3.2) is defined by the following equation:

$$f_3(x_i) = \frac{e^{x_i}}{\sum_{i=1}^{K} e^{x_i}}$$

wherein $x_i$ represents a weighted sum of the inputs of the $i$-$th$ node of the third hidden layer and $K$ is the total number of nodes of the third hidden layer (3.2).

In an embodiment, the plurality of disease models (5) comprises a dementia disease model (5) generated by the AI module (4). Figure 3c shows the final importance values of 13 health and personal parameters obtained with decision trees (9) for the dementia disease model (5) in an example. In this case, only the parameters above a predefined threshold of 0.03 are selected to train the AI module (4) for generating such dementia disease model (5):

- subject suffering a diabetes disease (Health_Conditions),
- subject suffering a depression disease (Depression_Status),
- subject with APOE $\varepsilon$3 allele (APOE_ $\varepsilon$3),
- level of oxygen in blood (BloodOxygenLevel),
- heart rate (HearRate),
- age,
- time elapsed between the subject wears a wearable device and a magnetic resonance imaging study is performed or between a magnetic resonance imaging study is performed and the subject puts on the wearable device (MRI_Deleay),
- level of alcohol in blood (AlcoholLevel),
- body temperature (BodyTemperature), and
- weight.

The parameters that are rejected are:

- medication history of the subject;
- family history; and
- sex.

In this embodiment, the selection of 13 health and personal parameters was performed using a random forest classifier, wherein all the possible health and personal parameters are used as input of said classifier.

Throughout all this document, "family history" will be understood as the health information about a subject's biological relatives; comprising parents, siblings, grandparents, aunts, uncles, and cousins. The family history is part of the medical history of a subject that provides valuable insights into a subject's risk of developing certain health conditions, as well as potential genetic predispositions or hereditary factors.

For this dementia disease model (5):

○ the activation function of each node of the first hidden layer (3.2) is defined by the following equation:

$$f_1(x) = \max(0, x)$$

wherein x represents a weighted sum of the inputs of each node of the first hidden layer (3.2);

∘ the activation function of each node of the second hidden layer (3.2) is defined by the following equation:

$$f_2(x) = x \cdot \tanh(\ln(1 + e^x))$$

wherein x represents a weighted sum of the inputs of each node of the second hidden layer (3.2); and

∘ the activation function of the third hidden layer (3.2) is defined by the following equation:

$$f_3(x) = \ln(1 + e^x)$$

wherein $x$ represents a weighted sum of the inputs of each node of the third hidden layer (3.2).

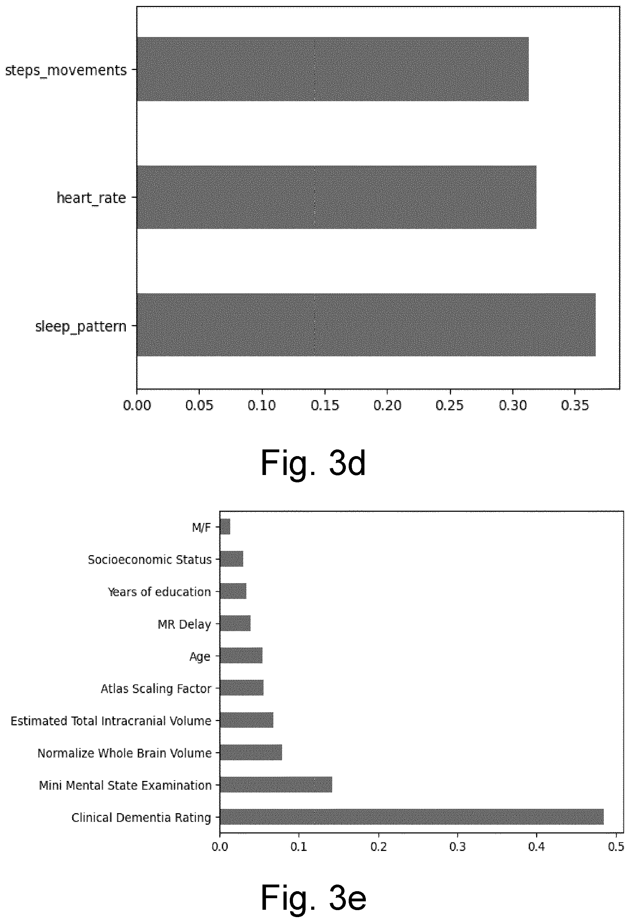

In an embodiment, the plurality of disease models (5) comprises a schizophrenia disease model (5) generated by the AI module (4). Figure 3d shows the final importance values of three health and personal parameters obtained with decision trees (9) for the schizophrenia disease model (5). In this case, the parameters above a predefined threshold of 0.25 are selected to train the AI module (4) for generating such schizophrenia disease model (5). In this case, all parameters are selected:

- sleep pattern,
- steps movements pattern, and
- heart rate.

In this embodiment, the selection of 3 health and personal parameters was performed using a random forest classifier, wherein all the possible health and personal parameters are used as input of said classifier.
For this schizophrenia disease model (5):

∘ the activation function of each node of the first hidden layer (3.2) is defined by the following equation:

$$f_1(x) = \max(0, x)$$

wherein $x$ represents a weighted sum of the inputs of each node of the first hidden layer (3.2);

∘ the activation function of each node of the second hidden layer (3.2) is defined by the following equation:

$$f_2(x) = \max(0, x)$$

wherein $x$ represents a weighted sum of the inputs of each node of the second hidden layer (3.2); and

∘ the activation function of the third hidden layer (3.2) is defined by the following equation:

$$f_3(x_i) = \frac{e^{x_i}}{\sum_{i=1}^{K} e^{x_i}}$$

wherein $x_i$ represents a weighted sum of the inputs of the $i$-$th$ node of the third hidden layer and $K$ is the total number of nodes of the third hidden layer (3.2).

In an embodiment, the plurality of disease models (5) comprises an Alzheimer disease model (5) generated by the AI module (4). Figure 3e shows the final importance values of 10 health and personal parameters obtained with decision trees (9) for the Alzheimer disease model (5). In this case, only the parameters above a predefined threshold of 0.05 are selected to train the AI module (4) for generating such Alzheimer disease model (5):

- clinical dementia rating, that indicates the severity of dementia symptoms,
- mini mental state examination, that indicates the degree of cognitive function,
- normalized whole brain volume,
- estimated total intracranial volume,
- atlas scaling factor, and
- age.

The parameters that are rejected are:

- time elapsed between the subject wears a wearable device and a magnetic resonance imaging study is performed or between a magnetic resonance imaging study is performed and the subject puts on the wearable device (MRI_Deleay),
- years of education of the subject;
- socioeconomic status of the subject; and
- sex.

In this embodiment, the selection of 10 health and personal parameters was performed using a random forest classifier, wherein all the possible health and personal parameters are used as input of said classifier.

For this Alzheimer disease model (5):

◦ the activation function of each node of the first hidden layer (3.2) is defined by the following equation:

$$f_1(x) = \max(0, x)$$

wherein x represents a weighted sum of the inputs of each node of the first hidden layer (3.2);

◦ the activation function of each node of the second hidden layer (3.2) is defined by the following equation:

$$f_2(x) = \max(0, x)$$

wherein $x$ represents a weighted sum of the inputs of each node of the second hidden layer (3.2); and

◦ the activation function of the third hidden layer (3.2) is defined by the following equation:

$$f_3(x_i) = \frac{e^{x_i}}{\sum_{i=1}^{K} e^{x_i}}$$

wherein $x_i$ represents a weighted sum of the inputs of the $i$-$th$ node of the third hidden layer and $K$ is the total number of nodes of the third hidden layer (3.2).

In an embodiment, the plurality of disease models (5) comprises an anxiety disease model (5) and/or a depression disease model (5) generated by the AI module (4) using the following parameters of subjects (2) that suffer from an anxiety disease and/or a depression disease:

- heart rate variability;
- electrodermal activity;
- respiratory rate and respiratory patterns;
- level of blood pressure;
- activity levels;
- sleep pattern;
- social interaction pattern;
- substance use or abuse history;
- self-reported symptoms of anxiety and/or depression;
- history of mental health diagnoses or treatments.

For these disease models (5):

◦ the activation function of each node of the first hidden layer (3.2) is defined by the following equation:

$$f_1(x) = \max(0, x)$$

wherein x represents a weighted sum of the inputs of each node of the first hidden layer (3.2);

◦ the activation function of each node of the second hidden layer (3.2) is defined by the following equation:

$$f_2(x) = \frac{1}{1 + e^{-x}}$$

wherein *x* represents a weighted sum of the inputs of each node of the second hidden layer (3.2); and

◦ the activation function of the third hidden layer (3.2) is defined by the following equation:

$$f_3(x) = \frac{e^x - e^{-x}}{e^x + e^{-x}}$$

wherein x represents a weighted sum of the inputs of each node of the third hidden layer (3.2).

In an embodiment, the plurality of disease models (5) comprises an eating disorder disease model (5) generated by the AI module (4) using the following parameters of subjects (2) that suffer from an eating disorder disease:

- age;
- sex;
- body mass index;
- number of binge episodes per week;
- number of purging episodes per week;
- body dissatisfaction score, that indicates the score of self-reported body dissatisfaction;
- eating disorder examination questionnaire score, that indicates the score of the eating disorder symptoms according to a standard;
- depression score, that indicates the score from a depression assessment scale;
- anxiety score, that indicates the score from an anxiety assessment scale; and
- eating disorder diagnosis.

For this eating disorder disease model (5):

◦ the activation function of each node of the first hidden layer (3.2) is defined by the following equation:

$$f_1(x) = \max(0, x)$$

wherein x represents a weighted sum of the inputs of each node of the first hidden layer (3.2);

◦ the activation function of each node of the second hidden layer (3.2) is defined by the following equation:

$$f_2(x) = \frac{1}{1 + e^{-x}}$$

wherein x represents a weighted sum of the inputs of each node of the second hidden layer (3.2); and

◦ the activation function of the third hidden layer (3.2) is defined by the following equation:

$$f_3(x) = \frac{e^x - e^{-x}}{e^x + e^{-x}}$$

wherein x represents a weighted sum of the inputs of each node of the third hidden layer (3.2).

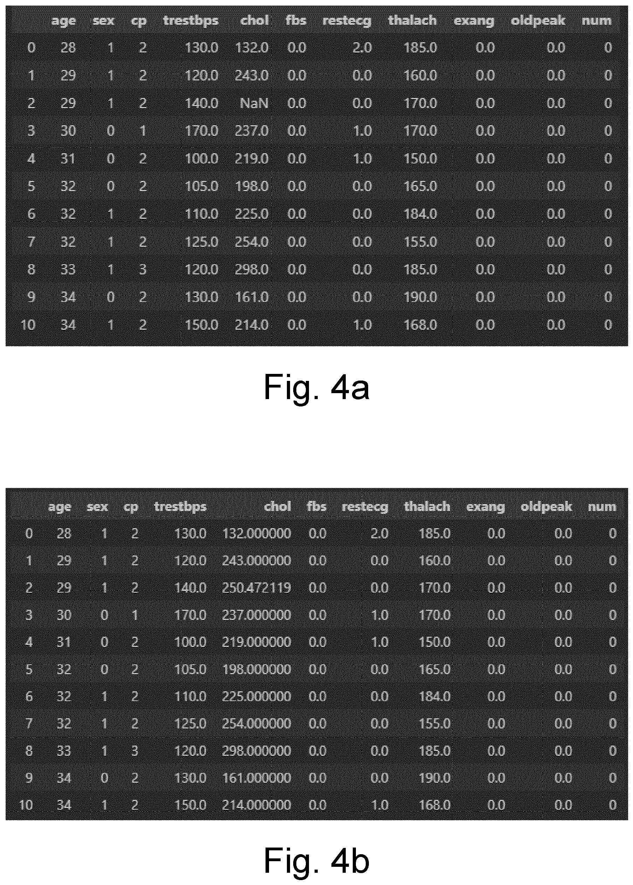

Figures 4a-4b show an example of table created with data from a medical database for training the AI module (4). In this specific example, the heart disease model (5) is being generated by the AI module (4).

In particular, the processor (3) is configured to process the health parameters and the personal parameters used to train the AI module (4) before the training process. Such data processing step carried out by the processor (3) requires the following steps:

- creating a table wherein each column represents a health parameter or a personal parameter and each row represents a different subject (2) suffering from the disease;
- giving a predetermined numerical format to the values of the table, in this case, integer numbers, what results in the table shown in Figure 4a;
- replacing the values of the table that do not present a numerical value (NaN) with the mean of its column's values, resulting in the table shown in Figure 4b;
- identifying the outlier values of each column; for example, using a Z-score algorithm; and
- eliminating the row or rows in which the outlier values are located.

Alternatively, the processor (3) is configured to carry out the processing of the health parameters and the personal parameters for each disease performing the following steps:

- creating a table wherein each row represents a health parameter or a personal parameter and each column represents a different subject suffering from the disease;
- giving a predetermined numerical format to the values of the table;
- replacing the values of the table that do not present a numerical value with the mean of its row's values;
- identifying the outlier values of each row, preferably using a Z-score algorithm; and
- eliminating the column or columns in which the outlier values are located.

## Claims

1. A system (1) for emitting alarms related to the health of a subject (2), the system (1) comprising a processor (3) configured to:

   - receive at least one health parameter of a subject, wherein one or more of the at least on health parameter is measured by at least one sensor (4), and
   - receive at least one personal parameter of the subject,
   wherein the processor (3) comprises an Artificial Intelligence, AI, module (4) trained to generate a plurality of disease models (5) as a result of the training, the training being carried out with:

      • at least one health parameter and/or at least one personal parameter of subjects (2) that suffer from at least one of the diseases of the disease models (5), and
      • at least one health parameter and/or at least one personal parameter of subjects (2) that do not suffer from said disease;

   wherein each disease model (5) is based on a neural network (6) comprising:

      ◦ an input layer (6.1) comprising N nodes, N being the number of health parameters and personal parameters used for the training of the disease model (5);
      ◦ three hidden layers (6.2), each one comprising an activation function and a plurality of nodes configured to receive a plurality of inputs; and
      ◦ an output layer (6.3) with a single node representing the probability of the subject to suffer from the disease; and

   wherein the output of each node of each hidden layer of the neural network(6) is the result of applying its corresponding activation function to a weighted sum of its corresponding inputs;
   and wherein the AI module (4) is configured to carry out the following steps:

      a) using the at least one health parameter and/or the at least one personal parameter as input for one or more of the disease models (5);
b) determining the probability of the subject to suffer from a disease; and
c) emitting an alarm (7) if the probability exceeds a determined probability value.

2. The system (1) according to claim 1, wherein the processor (3) is configured to select the health parameters and/or the personal parameters used to train the AI module (4) for generating each disease model (5), wherein the selection is carried out using a plurality of decision trees (9), each decision tree (9) comprising a plurality of layers (9.1, 9.2), and each layer (9.1, 9.2) comprising at least one parent node, at least one left child node and at least one right child node, and wherein the processor (3) is configured to carry out the following steps:

- using the health parameters and/or the personal parameters as the inputs of the plurality of decision trees (9);
- determining an importance value of each health parameter and each personal parameter for each layer (9.1, 9.2) of a decision tree as the decrease in node impurity weighted by the probability of reaching the at least one parent node of the layer (9.1, 9.2); wherein

  ◦ the probability of reaching the parent node of the layer (9.1, 9.2) is determined as the number of health parameters and personal parameters that reach said parent node divided by the total number of health parameters and personal parameters used to train the AI module (4); and
  ◦ the decrease in node impurity is determined as follows:

$$Decrease\ node\ impurity = I_p - \left( \frac{N_{left}}{N_{right}} \cdot I_{left} + \frac{N_{right}}{N_{left}} \cdot I_{right} \right)$$

being:

$N_{left}$ and $N_{right}$ the total number of health parameters and personal parameters in the at least one left and the at least one right child nodes of the layer (9.1, 9.2), respectively, and
$I_p$, $I_{left}$ and $I_{right}$ are, respectively, the impurities of the at least one parent node, the at least one left child node and the at least one right child node; the impurities being calculated with an impurity measure, preferably a Gini impurity measure or an entropy measure;

- determining a final importance value for each health parameter and each personal parameter as the importance value of said health parameter or personal parameter calculated for the last layer (9.2) of the decision tree (9); and
- selecting the health parameters and/or personal parameters to be used to train the AI module (4) as the ones with a final importance value over a predefined threshold.

3. The system (1) according to any of the preceding claims, wherein the processor (3) is configured to process the health parameters and/or the personal parameters used to train the AI module (4) before the training process for the disease models generation, the processor (3) being configured to carry out the processing of the health parameters and/or the personal parameters for each disease performing the following steps:

- creating a table wherein each column represents a health parameter or a personal parameter and each row represents a different subject suffering from the disease;
- giving a predetermined numerical format to the values of the table;
- replacing the values of the table that do not present a numerical value with the mean of its column's values;
- identifying the outlier values of each column, preferably using a Z-score algorithm; and
- eliminating the row or rows in which the outlier values are located;
or
- creating a table wherein each row represents a health parameter or a personal parameter and each column represents a different subject suffering from the disease;
- giving a predetermined numerical format to the values of the table;
- replacing the values of the table that do not present a numerical value with the mean of its row's values;
- identifying the outlier values of each row, preferably using a Z-score algorithm; and
- eliminating the column or columns in which the outlier values are located.

4. The system (1) according to any of the preceding claims, wherein the system (1) comprises at least one sensor (8.1), the at least one sensor (8.1) comprising a three axis gyroscope (8.1) and a three axis accelerometer (8.1) and wherein the AI module (4) is further configured for

- detecting if the subject (2) has fainted or has fallen down by analyzing the angular velocity measured by the three axis gyroscope (8.1) and the acceleration measured by the three axis accelerometer (8.1), and
- emitting an alarm (7) in case the subject (2) has fainted or has fallen down.

5. The system (1) according to any of the previous claims, wherein the system (1) comprises at least one sensor (8.1) configured for tracking the location of the subject (2) and the AI module (4) is further configured to emit an alarm (7) if the subject (2) is out of a predefined area.

6. The system (1) according to any of the previous claims, wherein the AI module (4) is further configured for:

- recommending a personalized treatment plan according to the probability of the subject (2) to suffer from a disease; and/or
- associating a subject (2) with at least one specific healthcare provider according to the probability of the subject (2) to suffer from a disease.

7. The system (1) according to any of the previous claims, wherein the AI module (4) is further configured for

- receiving at least one text and/or voice input from the subject (2);
- estimating the probability of the subject (2) to be following a specific emotional trend as follows:

$$P(A|B) = \frac{P(B|A) \cdot P(A)}{P(B)}$$

wherein:

○ B is the text and/or voice input from the subject (2);
○ A is the emotional trend;
○ *P(A)* is the prior probability that the subject (2) is following the emotional trend A;
○ *P(B)* is the prior probability of finding at least one predefined word in the text and/or voice input from the subject (2);
○ *P(B|A)* is the conditional probability of finding the at least one predefined word in the text and/or voice input from the subject if the subject (2) is following the emotional trend A; and
○ *P(A|B)* is the conditional probability that the subject (2) is following the emotional trend A if the at least one predefined word is found in the text and/or voice input from the subject (2); and

- comparing the estimated probability of the subject to be following a specific emotional trend with a predefined range of probabilities;
- detecting a health crisis if the estimated probability is within the predefined range; and
- emitting an alarm if a health crisis is detected.

8. The system (1) according to any of the previous claims, wherein the AI module (4) is configured for:

- receiving a medical history of a subject (2);
- estimating the probability of the subject (2) to be following a specific emotional trend as follows:

$$P(A|C) = \frac{P(C|A) \cdot P(A)}{P(C)}$$

wherein:

○ C is the medical history of the subject (2);
○ A is the emotional trend;
○ *P(A)* is the prior probability that the subject (2) is following the emotional trend A;
○ P(C) is the prior probability of finding at least one predefined data in the medical history of the subject (2);

○ *P(C|A)* is the conditional probability of finding the at least one predefined data in the medical history of the subject (2) if the subject (2) is following the emotional trend A; and
○ *P(A|C)* is the conditional probability that the subject (2) is following the emotional trend A if the at least one predefined data is found in the medical history of the subject (2); and

- comparing the estimated probability of the subject to be following a specific emotional trend with a predefined range of probabilities;
- detecting a health crisis if the estimated probability is within the predefined range; and
- emitting an alarm if a health crisis is detected.

9. The system (1) according to any of the preceding claims, wherein the system (1) comprises at least one wearable device (8) with at least one sensor (8.1) configured for:

- monitoring vital signs of the subject (2); and/or
- monitoring activity levels of the subject(2); and/or
- monitoring sleep patterns of the subject (2); and/or
- monitoring the heart rate of the subject (2); and/or
- monitoring the position of the subject (2); and/or
- measuring the blood pressure of the subject (2); and/or
- measuring the SpO2 of the subject (2); and/or
- measuring the temperature of the subject (2); and/or
- measuring the level of glucose in blood of the subject (2).

10. The system (1) according to any of the previous claims, wherein the alarm (7) comprises:

- a medical report to be sent to a healthcare provider; and/or
- an emergency call to the emergency services; and/or
- a notification to the subject and/or to a contact person and/or to a healthcare provider and/or to the emergency services.

11. The system (1) according to any of the preceding claims, wherein the plurality of disease models (5) comprises a heart disease model (5) generated by the AI module (4) using one or more of the following parameters of subjects (2) that suffer from a heart disease:

- age,
- type of chest pain experienced by the subject,
- level of blood pressure at resting mode,
- serum cholesterol,
- maximum heart rate experienced by the subject,
- angina induced by exercise, and
- ST-depression induced by exercise in comparison with the resting mode;

and wherein:

○ the activation function of each node of the first hidden layer (3.2) is defined by the following equation:

$$f_1(x) = \max(0, x)$$

wherein *x* represents a weighted sum of the inputs of each node of the first hidden layer (3.2);
○ the activation function of each node of the second hidden layer (3.2) is defined by the following equation:

$$f_2(x) = x \cdot \tanh(\ln(1 + e^x))$$

wherein *x* represents a weighted sum of the inputs of each node of the second hidden layer (3.2); and
○ the activation function of the third hidden layer (3.2) is defined by the following equation:

$$f_3(x_i) = \frac{e^{x_i}}{\sum_{i=1}^{K} e^{x_i}}$$

wherein $x_i$ represents a weighted sum of the inputs of the $i$ - $th$ node of the third hidden layer and $K$ is the total number of nodes of the third hidden layer (3.2).

**12.** The system (1) according to any of the preceding claims, wherein the plurality of disease models (5) comprises a dementia disease model (5) generated by the AI module (4) using one or more the following parameters of subjects (2) that suffer from a dementia disease (5):

- subject suffering a diabetes disease,
- subject suffering a depression disease,
- subject with APOE ε3 allele,
- level of oxygen in blood,
- heart rate,
- age,
- time elapsed between the subject wears a wearable device and a magnetic resonance imaging study is performed or between a magnetic resonance imaging study is performed and the subject puts on the wearable device,
- level of alcohol in blood,
- body temperature, and
- weight;

and wherein:

◦ the activation function of each node of the first hidden layer (3.2) is defined by the following equation:

$$f_1(x) = \max(0, x)$$

wherein x represents a weighted sum of the inputs of each node of the first hidden layer (3.2);
◦ the activation function of each node of the second hidden layer (3.2) is defined by the following equation:

$$f_2(x) = x \cdot \tanh(\ln(1 + e^x))$$

wherein x represents a weighted sum of the inputs of each node of the second hidden layer (3.2); and
◦ the activation function of the third hidden layer (3.2) is defined by the following equation:

$$f_3(x) = \ln(1 + e^x)$$

wherein x represents a weighted sum of the inputs of each node of the third hidden layer (3.2).

**13.** The system (1) according to any of the preceding claims, wherein the plurality of disease models (5) comprises a schizophrenia disease model (5) generated by the AI module (4) using one or more of the following parameters of subjects (2) that suffer from a schizophrenia disease:

- sleep pattern,
- steps movements pattern, and
- heart rate,

and wherein:

◦ the activation function of each node of the first hidden layer (3.2) is defined by the following equation:

$$f_1(x) = \max(0, x)$$

wherein *x* represents a weighted sum of the inputs of each node of the first hidden layer (3.2);

○ the activation function of each node of the second hidden layer (3.2) is defined by the following equation:

$$f_2(x) = \max(0, x)$$

wherein *x* represents a weighted sum of the inputs of each node of the second hidden layer (3.2); and

○ the activation function of the third hidden layer (3.2) is defined by the following equation:

$$f_3(x_i) = \frac{e^{x_i}}{\sum_{i=1}^{K} e^{x_i}}$$

wherein $x_i$ represents a weighted sum of the inputs of the *i - th* node of the third hidden layer and K is the total number of nodes of the third hidden layer (3.2).

**14.** The system (1) according to any of the preceding claims, wherein the plurality of disease models (5) comprises an Alzheimer disease model (5) generated by the AI module (4) using one or more of the following parameters of subjects (2) that suffer from an Alzheimer disease:

- clinical dementia rating, that indicates the severity of dementia symptoms;
- mini mental state examination, that indicates the degree of cognitive function;
- normalized whole brain volume,
- estimated total intracranial volume,
- atlas scaling factor, and
- age in years,

and wherein:

○ the activation function of each node of the first hidden layer (3.2) is defined by the following equation:

$$f_1(x) = \max(0, x)$$

wherein x represents a weighted sum of the inputs of each node of the first hidden layer (3.2);

○ the activation function of each node of the second hidden layer (3.2) is defined by the following equation:

$$f_2(x) = \max(0, x)$$

wherein x represents a weighted sum of the inputs of each node of the second hidden layer (3.2); and

○ the activation function of the third hidden layer (3.2) is defined by the following equation:

$$f_3(x_i) = \frac{e^{x_i}}{\sum_{i=1}^{K} e^{x_i}}$$

wherein $x_i$ represents a weighted sum of the inputs of the *i - th* node of the third hidden layer and K is the total number of nodes of the third hidden layer (3.2).

**15.** The system (1) according to any of the preceding claims, wherein the plurality of disease models (5) comprises an anxiety disease model (5) and/or a depression disease model (5) generated by the AI module (4) using one or more of the following parameters of subjects (2) that suffer from an anxiety disease and/or a depression disease:

- heart rate variability;
- electrodermal activity;
- respiratory rate and respiratory patterns;
- level of blood pressure;
- activity levels;

- sleep pattern;
- social interaction pattern;
- substance use or abuse history;
- self-reported symptoms of anxiety and/or depression;
- history of mental health diagnoses or treatments;

and wherein:

  ◦ the activation function of each node of the first hidden layer (3.2) is defined by the following equation:

$$f_1(x) = \max(0, x)$$

wherein x represents a weighted sum of the inputs of each node of the first hidden layer (3.2);
  ◦ the activation function of each node of the second hidden layer (3.2) is defined by the following equation:

$$f_2(x) = \frac{1}{1 + e^{-x}}$$

wherein x represents a weighted sum of the inputs of each node of the second hidden layer (3.2); and
  ◦ the activation function of the third hidden layer (3.2) is defined by the following equation:

$$f_3(x) = \frac{e^x - e^{-x}}{e^x + e^{-x}}$$

wherein $x$ represents a weighted sum of the inputs of each node of the third hidden layer (3.2).

16. The system (1) according to any of the preceding claims, wherein the plurality of disease models (5) comprises an eating disorder disease model (5) generated by the AI module (4) using one or more of the following parameters of subjects (2) that suffer from an eating disorder disease:

- age;
- sex;
- body mass index;
- number of binge episodes per week;
- number of purging episodes per week;
- body dissatisfaction score, that indicates the score of self-reported body dissatisfaction;
- eating disorder examination questionnaire score, that indicates the score of the eating disorder symptoms according to a standard;
- depression score, that indicates the score from a depression assessment scale;
- anxiety score, that indicates the score from an anxiety assessment scale; and
- eating disorder diagnosis;

and wherein:

  ◦ the activation function of each node of the first hidden layer (3.2) is defined by the following equation:

$$f_1(x) = \max(0, x)$$

wherein $x$ represents a weighted sum of the inputs of each node of the first hidden layer (3.2);
  ◦ the activation function of each node of the second hidden layer (3.2) is defined by the following equation:

$$f_2(x) = \frac{1}{1 + e^{-x}}$$

wherein *x* represents a weighted sum of the inputs of each node of the second hidden layer (3.2); and
∘ the activation function of the third hidden layer(3.2) is defined by the following equation:

$$f_3(x) = \frac{e^x - e^{-x}}{e^x + e^{-x}}$$

wherein *x* represents a weighted sum of the inputs of each node of the third hidden layer (3.2).

Fig.1a

Fig. 1b

Fig. 2

Fig. 3a

Fig. 3b

Fig. 3c

Fig. 3d

Fig. 3e

| | age | sex | cp | trestbps | chol | fbs | restecg | thalach | exang | oldpeak | num |
|---|---|---|---|---|---|---|---|---|---|---|---|
| 0 | 28 | 1 | 2 | 130.0 | 132.0 | 0.0 | 2.0 | 185.0 | 0.0 | 0.0 | 0 |
| 1 | 29 | 1 | 2 | 120.0 | 243.0 | 0.0 | 0.0 | 160.0 | 0.0 | 0.0 | 0 |
| 2 | 29 | 1 | 2 | 140.0 | NaN | 0.0 | 0.0 | 170.0 | 0.0 | 0.0 | 0 |
| 3 | 30 | 0 | 1 | 170.0 | 237.0 | 0.0 | 1.0 | 170.0 | 0.0 | 0.0 | 0 |
| 4 | 31 | 0 | 2 | 100.0 | 219.0 | 0.0 | 1.0 | 150.0 | 0.0 | 0.0 | 0 |
| 5 | 32 | 0 | 2 | 105.0 | 198.0 | 0.0 | 0.0 | 165.0 | 0.0 | 0.0 | 0 |
| 6 | 32 | 1 | 2 | 110.0 | 225.0 | 0.0 | 0.0 | 184.0 | 0.0 | 0.0 | 0 |
| 7 | 32 | 1 | 2 | 125.0 | 254.0 | 0.0 | 0.0 | 155.0 | 0.0 | 0.0 | 0 |
| 8 | 33 | 1 | 3 | 120.0 | 298.0 | 0.0 | 0.0 | 185.0 | 0.0 | 0.0 | 0 |
| 9 | 34 | 0 | 2 | 130.0 | 161.0 | 0.0 | 0.0 | 190.0 | 0.0 | 0.0 | 0 |
| 10 | 34 | 1 | 2 | 150.0 | 214.0 | 0.0 | 1.0 | 168.0 | 0.0 | 0.0 | 0 |

## Fig. 4a

| | age | sex | cp | trestbps | chol | fbs | restecg | thalach | exang | oldpeak | num |
|---|---|---|---|---|---|---|---|---|---|---|---|
| 0 | 28 | 1 | 2 | 130.0 | 132.000000 | 0.0 | 2.0 | 185.0 | 0.0 | 0.0 | 0 |
| 1 | 29 | 1 | 2 | 120.0 | 243.000000 | 0.0 | 0.0 | 160.0 | 0.0 | 0.0 | 0 |
| 2 | 29 | 1 | 2 | 140.0 | 250.472119 | 0.0 | 0.0 | 170.0 | 0.0 | 0.0 | 0 |
| 3 | 30 | 0 | 1 | 170.0 | 237.000000 | 0.0 | 1.0 | 170.0 | 0.0 | 0.0 | 0 |
| 4 | 31 | 0 | 2 | 100.0 | 219.000000 | 0.0 | 1.0 | 150.0 | 0.0 | 0.0 | 0 |
| 5 | 32 | 0 | 2 | 105.0 | 198.000000 | 0.0 | 0.0 | 165.0 | 0.0 | 0.0 | 0 |
| 6 | 32 | 1 | 2 | 110.0 | 225.000000 | 0.0 | 0.0 | 184.0 | 0.0 | 0.0 | 0 |
| 7 | 32 | 1 | 2 | 125.0 | 254.000000 | 0.0 | 0.0 | 155.0 | 0.0 | 0.0 | 0 |
| 8 | 33 | 1 | 3 | 120.0 | 298.000000 | 0.0 | 0.0 | 185.0 | 0.0 | 0.0 | 0 |
| 9 | 34 | 0 | 2 | 130.0 | 161.000000 | 0.0 | 0.0 | 190.0 | 0.0 | 0.0 | 0 |
| 10 | 34 | 1 | 2 | 150.0 | 214.000000 | 0.0 | 1.0 | 168.0 | 0.0 | 0.0 | 0 |

## Fig. 4b

Europäisches
Patentamt

European
Patent Office

Office européen
des brevets

## EUROPEAN SEARCH REPORT

**Application Number**

EP 24 17 6611

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2022/177728 A1 (UNIV PRINCETON [US]) 25 August 2022 (2022-08-25) | 1-10 | INV. G16H50/20 |
| A | * [0010], [0035], [0053], [0054], [0065], [0076], [0089], [0099], [0105], [0109] * | 11-16 | G16H50/30 G16H50/70 |
| | ----- | | |
| Y | WO 2022/251747 A1 (TEMPUS LABS INC [US]; GEISINGER CLINIC [US]) 1 December 2022 (2022-12-01) | 1-10 | |
| A | * [0023], [0118] * | 11-16 | |
| | ----- | | |
| Y | CN 117 789 973 A (ZHUHAI XINGZHI BIOTECHNOLOGY CO LTD) 29 March 2024 (2024-03-29) | 2 | |
| A | * [0012], [0129] * | 1,3-16 | |
| | ----- | | |
| Y | US 2023/078905 A1 (CHENG SHUCHUAN JACK [US] ET AL) 16 March 2023 (2023-03-16) | 4 | |
| A | * [0061], [0084] * | 1-3,5-16 | |
| | ----- | | |
| Y | US 2021/319894 A1 (SOBOL ADAM G [US] ET AL) 14 October 2021 (2021-10-14) | 5 | **TECHNICAL FIELDS SEARCHED (IPC)** |
| A | * [0021], [0109] * | 1-4,6-16 | G16H |
| | ----- | | |
| A | JP 2018 120394 A (RICOH CO LTD) 2 August 2018 (2018-08-02) * [0027], [0125] * | 1-16 | |
| | ----- | | |

The present search report has been drawn up for all claims

| Place of search | Date of completion of the search | Examiner |
|---|---|---|
| Munich | 17 October 2024 | Sundqvist, Benjamin |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 17 6611

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2024

| Patent document cited in search report | | Publication date | Patent family member(s) | | Publication date |
|---|---|---|---|---|---|
| WO 2022177728 | A1 | 25-08-2022 | EP | 4295278 A1 | 27-12-2023 |
| | | | WO | 2022177728 A1 | 25-08-2022 |
| WO 2022251747 | A1 | 01-12-2022 | AU | 2022280964 A1 | 21-12-2023 |
| | | | AU | 2022281440 A1 | 14-12-2023 |
| | | | BR | 112023024902 A2 | 20-02-2024 |
| | | | CA | 3220416 A1 | 10-12-2022 |
| | | | CA | 3220417 A1 | 01-12-2022 |
| | | | EP | 4346564 A1 | 10-04-2024 |
| | | | EP | 4346565 A1 | 10-04-2024 |
| | | | EP | 4346566 A1 | 10-04-2024 |
| | | | JP | 2024522121 A | 11-06-2024 |
| | | | US | 2022384044 A1 | 01-12-2022 |
| | | | US | 2022384045 A1 | 01-12-2022 |
| | | | US | 2023343464 A1 | 26-10-2023 |
| | | | WO | 2022251747 A1 | 01-12-2022 |
| | | | WO | 2022251748 A1 | 01-12-2022 |
| | | | WO | 2022251750 A1 | 01-12-2022 |
| CN 117789973 | A | 29-03-2024 | NONE | | |
| US 2023078905 | A1 | 16-03-2023 | US | 2023042243 A1 | 09-02-2023 |
| | | | US | 2023078905 A1 | 16-03-2023 |
| US 2021319894 | A1 | 14-10-2021 | US | 2021319894 A1 | 14-10-2021 |
| | | | US | 2024274285 A1 | 15-08-2024 |
| JP 2018120394 | A | 02-08-2018 | NONE | | |

EPO FORM P0459

For more details about this annex : see Official Journal of the European Patent Office, No. 12/82